(19) Europäisches Patentamt
European Patent Office
Office européen des brevets

(11) Publication number: **0 054 862**
**B1**

(12) # EUROPEAN PATENT SPECIFICATION

(45) Date of publication of patent specification: **27.11.85**

(21) Application number: **81110337.3**

(22) Date of filing: **11.12.81**

(51) Int. Cl.⁴: **C 07 K 5/06,** C 07 C 103/18, C 07 C 133/02, C 07 F 9/30, C 07 F 9/38, A 61 K 37/02

(54) **Substituted dipeptides, processes for their preparation and pharmaceutical compositions containing them and their use in the inhibition of enkephalinase.**

(30) Priority: **18.12.80 US 217621**
**28.04.81 US 258485**
**12.02.81 US 233548**
**12.02.81 US 233549**
**28.04.81 US 258486**
**28.04.81 US 258487**

(43) Date of publication of application:
**30.06.82 Bulletin 82/26**

(45) Publication of the grant of the patent:
**27.11.85 Bulletin 85/48**

(84) Designated Contracting States:
**AT BE CH DE FR GB IT LI NL SE**

(56) References cited:
**EP-A-0 009 183**
**EP-A-0 012 401**

(73) Proprietor: **SCHERING CORPORATION**
**2000 Galloping Hill Road**
**Kenilworth, New Jersey 07033 (US)**

(72) Inventor: **Berger, Joel G.**
**54 Park Avenue**
**Verona, New Jersey 07044 (US)**
Inventor: **Weinstein, Jay**
**191 Bellevue Avenue**
**Upper Montclair, New Jersey 07043 (US)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 054 862 B1**

Courier Press, Leamington Spa, England.

**Description**

This invention relates to certain novel substituted dipeptides and processes for their preparation. The invention also relates to pharmaceutical compositions containing substituted dipeptides including novel dipeptides in accordance with this invention.

A natural opiate receptor agonist, known as "enkephalin", which is believed to be a mixture of two peptides H-Tyr-Gly-Gly-Phe-Met-OH (methionine-enkephalin), and H-Tyr-Gly-Gly-Phe-Len-OH (leucine-enkephalin), both being subsumed hereinafter under the generic name "enkephalin", has been reported to produce a profound analgesia in rats, when injected into the brain ventricles thereof, (Beluzzic et al Nature 260, 625 (1976)).

It is known that enkephalin, naturally produced in the body of warm-blooded animals is inactivated by enzymes of the group known as enkephalinases which are also naturally produced in such bodies. It is therefore, of interest to find compounds capable of inhibiting or mitigating the above-mentioned inactivating effect of the enkephalinases.

One aspect of the present invention provides substituted dipeptides of the formula:

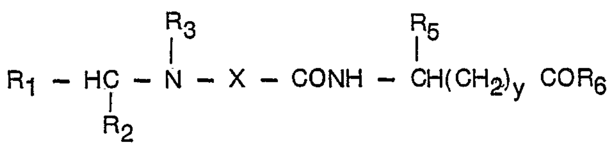

I

and the pharmaceutically acceptable salts thereof, in which

$R_1$ is hydrogen; alkyl; halogen loweralkyl; hydroxy loweralkyl; loweralkoxy loweralkyl; aryloxy loweralkyl; amino loweralkyl; loweralkylamino loweralkyl; di-loweralkyl aminoloweralkyl; acylamino loweralkyl; diarylaminoloweralkyl; arylamino loweralkyl; guanidino loweralkyl; heteroaryl; aryl, aralkyl; mercapto loweralkyl; arylthio loweralkyl; loweralkylaralkyl; alkylthio loweralkyl; aralkyloxyalkyl; aralkylthioalkyl; or heteroaryloxyalkyl; wherein the latter 3 radicals may be substituted with halogen, loweralkyl, loweralkoxy, hydroxy, amino, aminomethyl, carboxyl, cyano and/or sulfamoyl; or alkenyl (preferably loweralkenyl) substituted by a heterocyclic (e.g. heteroaryl) group or alkyl (e.g. loweralkyl) substituted by a heterocyclic (e.g. heteroaryl) group, the latter two groups optionally being substituted by one or more groups chosen from loweralkyl, hydroxy, loweralkoxy, amino, loweralkylamino, di-loweralkylamino, acylamino, halogeno, halogenoloweralkyl, cyano and/or sulfonamide;

$R_2$ is —COOH, COO-(loweralkyl), —COO-(aryl lower alkyl), —COO aryl, or the group

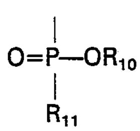

wherein

$R_{10}$ is hydrogen, lower alkyl or benzyl; and

$R_{11}$ is hydroxy, alkoxy, benzyloxy, lower alkyl, aryl or benzyl;

$R_3$ is hydrogen or lower alkyl;

X is 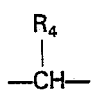 or 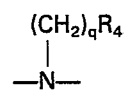

wherein q is 0 or 1;

$R_4$ and $R_5$ are chosen from the groups defined for $R_1$, indolyl (e.g. 3-indolyl), indolylalkyl (e.g. 3-indolylalkyl), adamantyl (e.g. 1-adamantyl), adamantylmethyl (e.g. 1-adamantylmethyl) amino methylphenylloweralkyl;

y is zero or an integer from 1 to 3;

$R_6$ is chosen from hydroxy, loweralkoxy and the group $—NR_8R_9$ in which $R_8$ and $R_9$ are independently hydrogen, aralkyl, or loweralkyl which together with the nitrogen atom to which they are attached may form a 4 to 6 membered heterocyclic group, one of whose members may be oxygen or sulphur;

and wherein, when X is

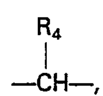,

and y is zero, the following provisions apply:

(i) if $R_2$ is —COOH, —COO(loweralkyl), —COO(arylloweralkyl) or —COO(aryl), and $R_6$ is OH, lower-alkoxy, alkylamino, dialkylamino, aralkylamino or diarylalkylamino, then $R_4$ is chosen from G—A— in which G is 2- or 3-thienyl or 1- or 2-naphthyl and A is —$CH_2$— or —$CH_3$-loweralkyl-,

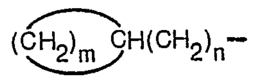

in which n is 0 or an integer from 1 to 8 and m is an integer from 2 to 8, adamantyl-methyl, unsubstituted or substituted phenyl, or substituted phenylloweralkyl, in which the substituent(s) for the latter two groups is or are chosen from halogen, trifluoromethyl, nitro, loweralkyl and loweralkoxy; further provided that

(ii) if, when $R_2$ and $R_6$ are as set forth in proviso (i), and $R_1$ is hydrogen or methyl, $R_3$ is hydrogen and $R_5$ is hydrogen, lower alkyl, unsubstituted phenyl, benzyl, benzyl substituted by halogen, hydroxyloweralkyl, loweralkoxyloweralkyl, phenoxy loweralkyl, mercapto loweralkyl, alkylthioloweralkyl, phenylthioloweralkyl or benzyl lower alkyl, then $R_4$ is chosen from G—A as defined above,

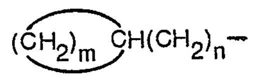

as defined above, (but excluding cyclohexyl and cyclohexylmethyl) adamantyl methyl, phenyl, or phenyl loweralkyl which latter two groups are substituted by nitro, loweralkyl or lower alkoxy;

(iii) if, when $R_1$, $R_2$, $R_3$ and $R_5$ are as set forth in proviso (ii) and $R_6$ is amino then $R_4$ cannot be the group —$(CH_2)_t$—$R_x$ in which t is 0 or 1 and $R_x$ is hydrogen, alkyl, cyclohexyl, phenyl or phenyl substituted by halogen, trifluoromethyl or hydroxy;

(iv) when $R_6$ is amino, $R_1$ is hydrogen, alkyl $C_1$—$C_{10}$, hydroxy-lower-alkyl, lower-alkoxy-lower alkyl, aryloxy-lower-alkyl, heteroaryloxy-loweralkyl, amino-lower-alkyl, loweralkylaminoloweralkyl, diloweralkylamino-lower alkyl, acylaminoloweralkyl, arylaminoloweralkyl, guanidino-lower-alkyl, alkyl substituted by a heterocyclic group, alkylthioloweralkyl, arylthioloweralkyl, arylloweralkyl, aralkyloxyloweralkyl, aralkylthioloweralkyl, in which the aryl group of any foregoing aryl containing substituent may be substituted by halogen, loweralkyl, hydroxy, lower-alkoxy-amino, cyano or sulfonamido; $R_2$ is —COOH, —COO alkyl, COO aryl, —COO (arylloweralkyl)), $R_3$ is hydrogen, and $R_4$ is hydrogen, lower-alkyl, phenyl-lower-alkyl, amino-methylphenyllower-alkyl, hydroxy-phenylloweralkyl, hydroxyloweralkyl, acylaminoloweralkyl, aminoloweralkyl, dimethyl aminoloweralkyl, guanidinoloweralkyl, indolylalkyl, lower-alkylthio-lower-alkyl; then $R_5$ cannot be a group having the structure

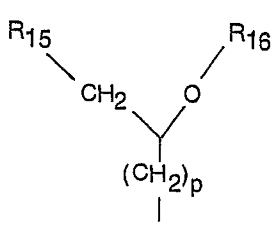 ; 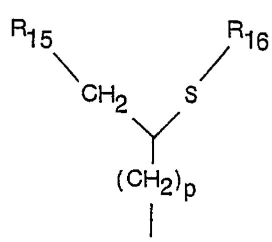 ; 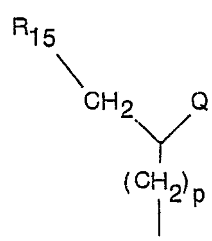 ;

wherein p is 0, 1 or 2

$R_{15}$ is hydrogen or lower alkyl

$R_{16}$ is lower alkyl, aryl, aralkyl or heteroaryl and

Q is $C_3$—$C_8$ cycloalkyl; or the group

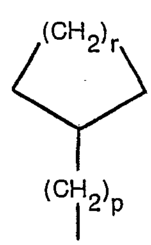

wherein p is as defined above and r is 2 or 3.

One sub-class of compounds which is of interest is that in which:

X is 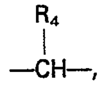

y is 1, 2 or 3, and $R_2$ is —COOH, —COO(loweralkyl), —COO(arylloweralkyl), or —COO(aryl), $R_3$ is hydrogen; $R_4$ is chosen from hydrogen, the group Th-$CH_2$-B-, as defined above, the group Naph-$CH_2$-B- as defined above, 1-adamantyl methyl, cycloloweralkyl, hydroxyloweralkyl, acylaminoloweralkyl, (e.g. acetylamino-loweralkyl), aminoloweralkyl, diloweralkylamino loweralkyl, halogeno loweralkyl, guanidino loweralkyl, imadazolyl loweralkyl, indolylloweralkyl, mercaptoloweralkyl, loweralkylthioloweralkyl, unsubstituted and substituted phenyl, and unsubstituted and substituted phenylloweralkyl in which the substituent(s) of the latter two groups is/are chosen from halogeno, loweralkyl, lower alkoxy, trifluoromethyl and/or nitro, the group

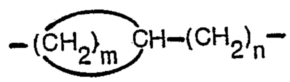

as defined above, and aminomethylphenylloweralkyl.

Another sub-class of compounds which is of interest is that in which:

X is 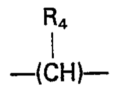

and $R_2$ is —COOH, —COO(loweralkyl), —COO(aryllower alkyl), or —COO(aryl) and y is 0,

$R_1$ is H, alkyl, aralkyl, phenyl, aralkyloxyalkyl, aralkylthioalkyl or heteroaryloxyalkyl, the latter three radicals optionally being substituted as defined above;

$R_3$ is hydrogen;

$R_4$ is chosen from G—A— as defined above

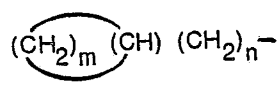

as defined above substituted or unsubstituted phenyl or substituted phenylloweralkyl in which the substituent(s) for the latter two groups is or are chosen from halogen, trifluoromethyl, nitro, loweralkyl, and loweralkoxy, with the proviso for $R_4$ only that when $R_6$ is the group

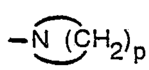 or 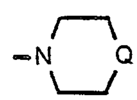

in which p is 3, 4 or 5, and Q is oxygen or sulfur, $R_4$ may also be hydrogen, loweralkyl, indolylloweralkyl, mercaptoloweralkyl, loweralkylthio-loweralkyl, guanidinoloweralkyl, imidazolyl-loweralkyl, hydroxy substituted phenyl and

$R_5$ is chosen from the groups defined for $R_4$ above, including those set forth in the proviso.

$R_6$ is chosen from hydroxy, loweralkoxy or the group $—NR'_8R'_9$, wherein $R'_8$ and $R'_9$ independently are chosen from hydrogen, loweralkyl, or together with the nitrogen atom to which they are attached are

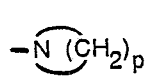 or 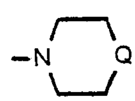

in which p and Q are as defined above.

Another sub-class of compounds of interest is that in which X is

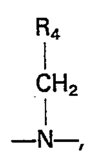

y is preferably zero,

$R_1$ is as defined above with reference to formula I, with the possible exception of alkenyl substituted by a heterocyclic group and alkyl substituted by a substituted heterocyclic group.

$R_2$ is —COO(aryl), —COO(aralkyl) or preferably —COOH or —COO(loweralkyl);

$R_3$ is hydrogen or loweralkyl;

$R_4$ is preferably one of the groups defined for $R_1$ in this group of compounds or

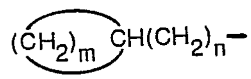

as defined above, adamantyl or indolyl,

$R_5$ is preferably one of the groups defined for $R_4$ in this group of compounds, and $R_6$ is preferably —OH.

Yet another sub-class of compounds of interest is that in which $R_2$ is

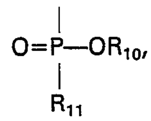

y is preferably zero,

$R_1$ is as defined above with reference to formula I with the possible exception of alkenyl substituted by a heterocyclic group and alkyl substituted by a substituted heterocyclic group.

X is 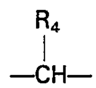 or 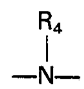

$R_3$, $R_4$, $R_5$, $R_{10}$ and $R_{11}$ are as defined above with reference to formula I; $R_2$ is —COOH or —COO(loweralkyl);

$R_6$ is —OH; and wherein, when $R_4$ is hydrogen, aryl or heteroaryl X is preferably

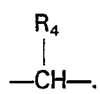

In this Specification the following definitions apply:

1) The terms alkyl, alkoxy and alkenyl signify such groups having from 1 to 20 carbon atoms and include such groups which are straight chained, or branched chained and/or comprise one or more alicyclic rings;

2) The terms lower alkyl, lower alkoxy and lower alkenyl signify such groups having 1 to 8 carbon atoms;

3) The term aralkyl signifies such groups having 7 to 20 carbon atoms in which the alkyl moiety may be straight chained or branched chained and/or comprise one or more alicyclic rings;

4) Heterocyclic — embraces groups having a 4, 5 or 6 member ring in which two of the members may be hetero atoms, one of which is nitrogen and the other is oxygen or sulfur;

5) Heteroaryl — embraces 5 or 6 member aromatic rings having 1 or 2 hetero atoms chosen from S, N and O.

6) Heteroaralkyl — signifies alkyl groups as defined above substituted by heteroaryl as defined above;

7) Aryl — denotes a radical derived from an aromatic hydrocarbon by the removal of one hydrogen atom and embraces phenyl, tolyl and naphthyl, and substituted phenyl wherein the substituents are preferably chosen from halogen, hydroxy, trifluoromethyl, nitro, loweralkoxyamino, loweralkylamino, di-loweralkylamino, acylamino, loweralkyl, loweralkoxy, amino, cyano and/or sulfanamide.

Among those novel compounds defined by formula I, in which X is

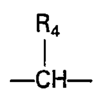 and $R_2$ is other than 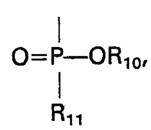

and y is preferably zero,
some which are of interest are, for example, those in which $R_4$ is chosen from 1-adamantyl, 1- or 2-naphthyl methyl, 2- or 3-thienyl methyl, halogeno (e.g. fluoro) substituted benzyl, and cyclopropyl-methyl; also those in which $R_1$ is phenyl lower-alkyl e.g. benzyl or phenyl-ethyl, or lower-alkyl, e.g. methyl or ethyl, particularly when $R_4$ is one of the groups mentioned immediately above; and also those in which $R_5$ is

hydrogen or methyl, particularly when $R_1$ and $R_4$ are members of the groups defined immediately above; and also those in which $R_2$ is —COOH or —COO(lower alkyl), e.g. —$COOCH_3$ or —$COOC_2H_5$, particularly when $R_1$, $R_4$ and $R_5$ are members of the groups set forth immediately above, and also those in which $R_6$ is hydroxy or lower alkoxy, particularly when $R_1$, $R_2$, $R_4$ and $R_5$ are members of the group set forth immediately above.

Representative of such novel compounds are the following:

N-(L-1-ethoxycarbonylethyl)-L-2-thienyl-alanylglycine,
N-(L-1-ethoxycarbonylethyl)-L-2-thienyl-alanyl-L-alanine,
N-(L-1-ethoxycarbonylethyl)-L-3-thienyl-alanyl-L-alanine,
N-(L-1-ethoxycarbonylethyl)-L-3-thienyl-alanylglycine,
N-(L-1-carboxyethyl)-L-2-thienylalanyl-L-alanine,
N-(L-1-tert-butoxycarbonylethyl)-L-2-thienylalanyl-L-alanine,
N-(L-1-carboxy-3-phenylpropyl)-L-3-(3-thienyl)alanyl-L-alanine m.p. 192.5°C—193°C;
N-(D,L-1-carboxy-3-phenylpropyl)-L-3-(2-naphthyl)alanyl-L-alanine m.p. 149°C—155°C;
N-(L-1-ethoxycarbonyl-3-phenylpropyl)-L-3-(2′-thienyl)alanyl-L-alanine, or the maleate salt thereof m.p. 124°C—124.5°C;
N-(D-1-carboxy-3-phenylpropyl)-L-(4-fluorophenyl)alanyl-L-alanine m.p. 147°C—152°C;
N-(L-1-carboxy-3-phenylpropyl)-L-3-(1-naphthyl)alanyl-β-alanine;
N-(D-1-carboxy-3-phenylpropyl)-L-3-(3-thienyl)alanyl-L-alanine m.p. 163°C—164°C;
N-(L-1-carboxy-2-(benzylthio)-ethyl)-L-phenylalanyl-β-alanine;
N-(L-1-carboxy-2-phenethyl)-L-phenylalanyl-β-alanine m.p. 218°C—219°C;
N-(L-1-carboxy-2-phenethyl)-L-phenylalanyl-γ-aminobutyric acid m.p. 209°C—211°C;
N-(L-1-carboxy-3-phenylpropyl)-L-phenylalanyl-β-alanine hemihydrate m.p. 176°C—190°C;
N-(L-1-carboxy-3-phenylpropyl)-D,L-3-(1-naphthyl)alanyl-L-alanine m.p. 186°C—194°C

and the pharmaceutically acceptable salts thereof.

Among the compounds of formula I in which X is

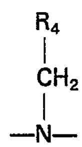

and $R_2$ is carboxy or —COO(loweralkyl), $R_6$ is OH and y is preferably zero, which are of interest are e.g. those in which
$R_1$ and $R_3$ are both hydrogen and
$R_2$ is —COO(loweralkyl) e.g. —$COOC_2H_5$;
also those compounds in which
$R_1$ is hydrogen, alkyl or aralkyl,
$R_4$ is 3-indolyl, aryl or heteroaryl,
$R_5$ is hydrogen or loweralkyl, and
$R_3$ is hydrogen or loweralkyl;
also those compounds in which
$R_1$ is aralkyl e.g. $C_6H_5$—$(CH_2)_2$—,
$R_2$ is carboxy,
$R_4$ is aryl e.g. phenyl, and
$R_3$ and $R_5$ are both hydrogen;
also those compounds in which
$R_1$ is alkyl e.g. methyl,
$R_2$ is carboxy,
$R_3$ is hydrogen,
$R_4$ is heteroaryl e.g. 4-imidazolyl, and
$R_5$ is loweralkyl e.g. methyl,
and those in which
$R_1$ is methyl, phenethyl or aralkthioalkyl,
$R_2$ is —COOH,
$R_4$ is phenyl and
$R_5$ is hydrogen, methyl or isobutyl; and
those compounds in which
$R_1$ is phenethyl or methyl,
$R_2$ is —COO(loweralkyl),
$R_3$ is hydrogen,

$R_4$ is 3-indolyl, heteroaryl or aryl, and

$R_5$ is hydrogen or lower alkyl.

Representative examples of such compounds are those having the following combinations of substituents:

(i) $R_1$ = hydrogen,
$R_2$ = —$COOC_2H_5$,
$R_3$ = H,
$R_4$ = 3-indolyl,
$R_5$ = H; and

(ii) $R_1$ = methyl,
$R_2$ = —COOH,
$R_3$ = H
$R_4$ = 4-imidazolyl,
$R_5$ = methyl.

Among the compounds of formula I in which $R_2$ is

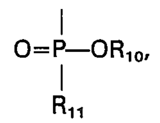

$R_6$ is —OH and y is preferably zero,
which are generally of interest when X is

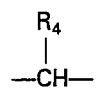

are those in which $R_1$ is hydrogen, alkyl, aryl or aralkyl and $R_4$ is lower alkyl, aryl, aralkyl (e.g. benzyl), heteroalkyl or heteroaryl, for instance such compounds in which $R_1$ is methyl or phenethyl, $R_3$ is hydrogen, $R_5$ is hydrogen or lower alkyl (e.g. methyl), $R_{10}$ is hydrogen and $R_{11}$ is hydroxy or methoxy; and those which are generally of interest when X is

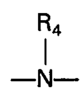

are those in which $R_1$ is hydrogen, alkyl, aryl and aralkyl and $R_4$ is lower alkyl, aralkyl e.g. benzyl or heteroaralkyl, for instance such compounds in which $R_1$ is methyl or phenethyl, $R_3$ is hydrogen and $R_5$ is hydrogen or lower alkyl e.g. methyl and $R_{10}$ is hydrogen and $R_{11}$ is hydroxy or methoxy.

Another group of compounds of particular interest comprises compounds in which $R_1$ is methyl or phenethyl, $R_4$ is benzyl, $R_5$ is hydrogen or methyl $R_{10}$ is hydrogen and $R_{11}$ is methyl and X is

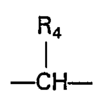 or 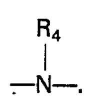.

We have found that the compounds as defined above with reference to Formula 1 including those excluded by the proviso (i) but excluding those compounds excluded by provisos (ii), (iii) and (iV) are useful in inhibiting enkephalinase, as described with reference to the *in vivo* and *in vitro* tests below. Representative exmaples of such compounds, in addition to those given above are as follows:

N-(L-1-carboxy-3-phenylpropyl)-L-phenylalanyl-L-alanine,
N-(D,L-1-carboxy-ethyl)-L-phenylalanyl-L-alanine,
N-(D,L-carboxy-3-phenylpropyl)-L-phenyl-alanyl glycine,
N-(L-1-carboxy-3-phenylpropyl)-L-phenyl-alanyl-L-alanine, and
N-(L-carboxy-2-methyl propyl)-D,L-phenyl-alanyl-L-alanine,
N-(L-1-ethoxycarbonyl-3-phenylpropyl)-D-phenylalanyl-L-alanine

and pharmaceutically acceptable salts thereof.

The compounds of the present invention can be prepared by a number of methods as described below.

a) One method, broadly speaking, (for producing compounds in which $R_2$ is other than

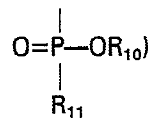

comprises reducing a compound having the formula:

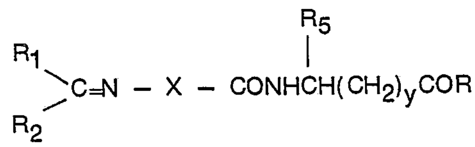

A

in which $R_1$, $R_2$ and $R_6$ may include suitable protection of any reactive groups, followed by removal of any protecting groups, if necessary, so as to provide a dipeptide of the formula:

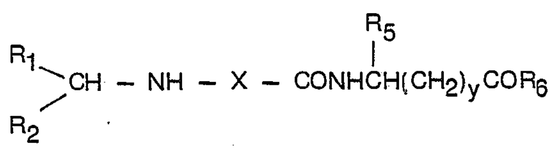

B

and, if desired, preferably when X is

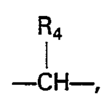

when $R_6$ is —OH in the resulting compound of formula *B*, converting $R_6$ to an alkoxy group or to the group $—NR_8R_9$.

The above method may be carried out by reducing a Schiff's base as it is formed in a reaction mixture from a ketone acid or ester and an amino acid or ester according to the overall reaction.

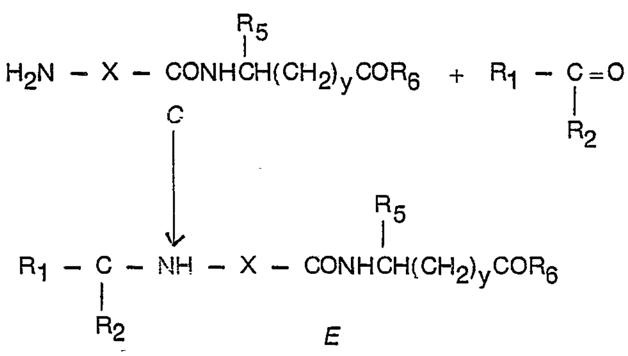

C

D

E

In the above reductive condensation reaction X is preferably

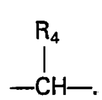

For example, the reaction between the keto acid or ester and the primary amine *C* can be carried out in solution using for example water or acetonitrile as solvent under substantially neutral conditions in the presence of a suitable reducing agent, for example sodium cyanoborohydride.

Alternatively a Schiff's base obtained by the reaction of the keto acid or ester *C* and the amino acid or ester *D* may be catalytically reduced in the presence of hydrogen at 1—4 atmospheres; suitable catalysts are for example Raney nickel and palladium on carbon e.g. 10% Pd on carbon.

Usually the group $COR_6$ will be a protected carboxyl group e.g. benzyloxycarbonyl or loweralkoxycarbonyl e.g. t-butyloxycarbonyl. In such instances the protecting group can be reduced giving a compound of formula I in which $R_6$ is hydroxy, using conventional hydrolysis or hydrogenolysis procedures, for example hydrolysis under basic conditions, for instance by reaction with sodium hydroxide in a suitable solvent.

When $R_6$ is —OH it may be converted to the group $—NR_8R_9$ or to an alkoxy group by conventional procedures. For example, a compound of formula I may be reacted with $SOCl_2$ or oxalyl chloride to convert the $R_6$ group into an activated ester group, and thereafter reacted with an amine $HNR_8R_9$ or alcohol to obtain the desired compounds.

Compound *C* can be prepared by following the reaction sequence given below (the compounds in which X is

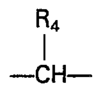

are exemplified).

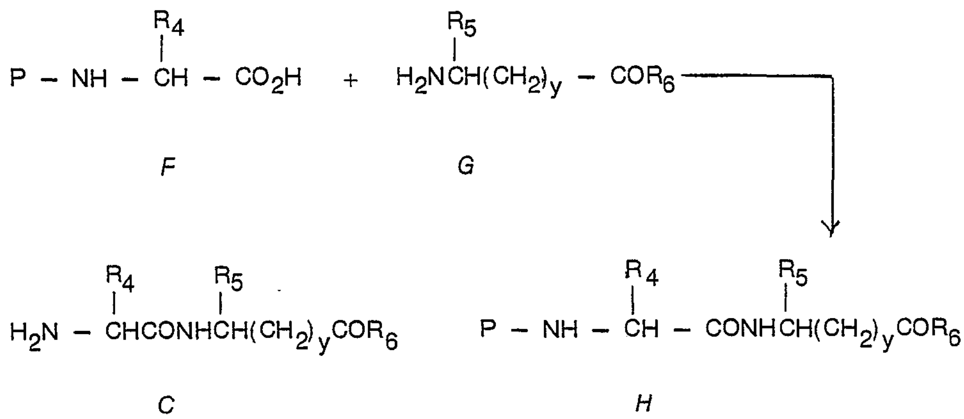

*F* *G*

*C* *H*

In the above reaction sequence the amino group of the amino acid *F* may be protected e.g. by any conventional amino protecting group P, for e.g. benzyloxycarbonyl or t-butyloxy carbonyl.

The amino acid *F* can be condensed with an amino ester derivative *G* for example using a condensing agent such as dicyclohexylcarbodiimide (DCC) or diphenylphosphoryl azide. An activating agent, for example 1-hydrodybenzothiazole, may be employed in the condensation reaction.

The amino protecting group *P* attached to the resulting dipeptide *H* is then removed by conventional treatment, for example, by treatment with an acid or by hydrogenation, for example, using hydrogen in the presence of a metal catalyst.

In the case of those compounds in which X is

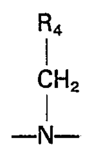

the reduction of the compound *A* may for example be carried out after isolation of the base of formula:

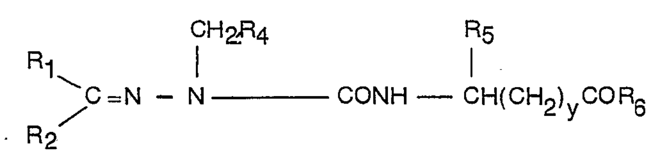

T

using a reducing agent e.g. sodium cyanoborohydride under weakly acidic conditions. The resulting compound of the formula

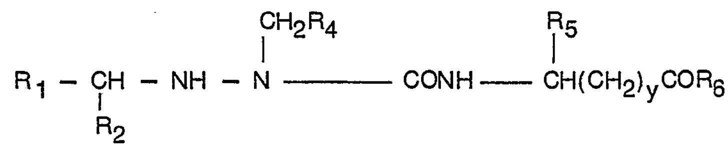

can then be treated, as required to convert $R_6$, if it is a carboxy protecting group, by well-known methods such as hydrolysis or hydrogenation to yield a terminal carboxy group, and likewise $R_2$, if it is carboxy protected can be converted, if desired, to carboxyl.

The starting compound T can be formed by the following reaction sequence:

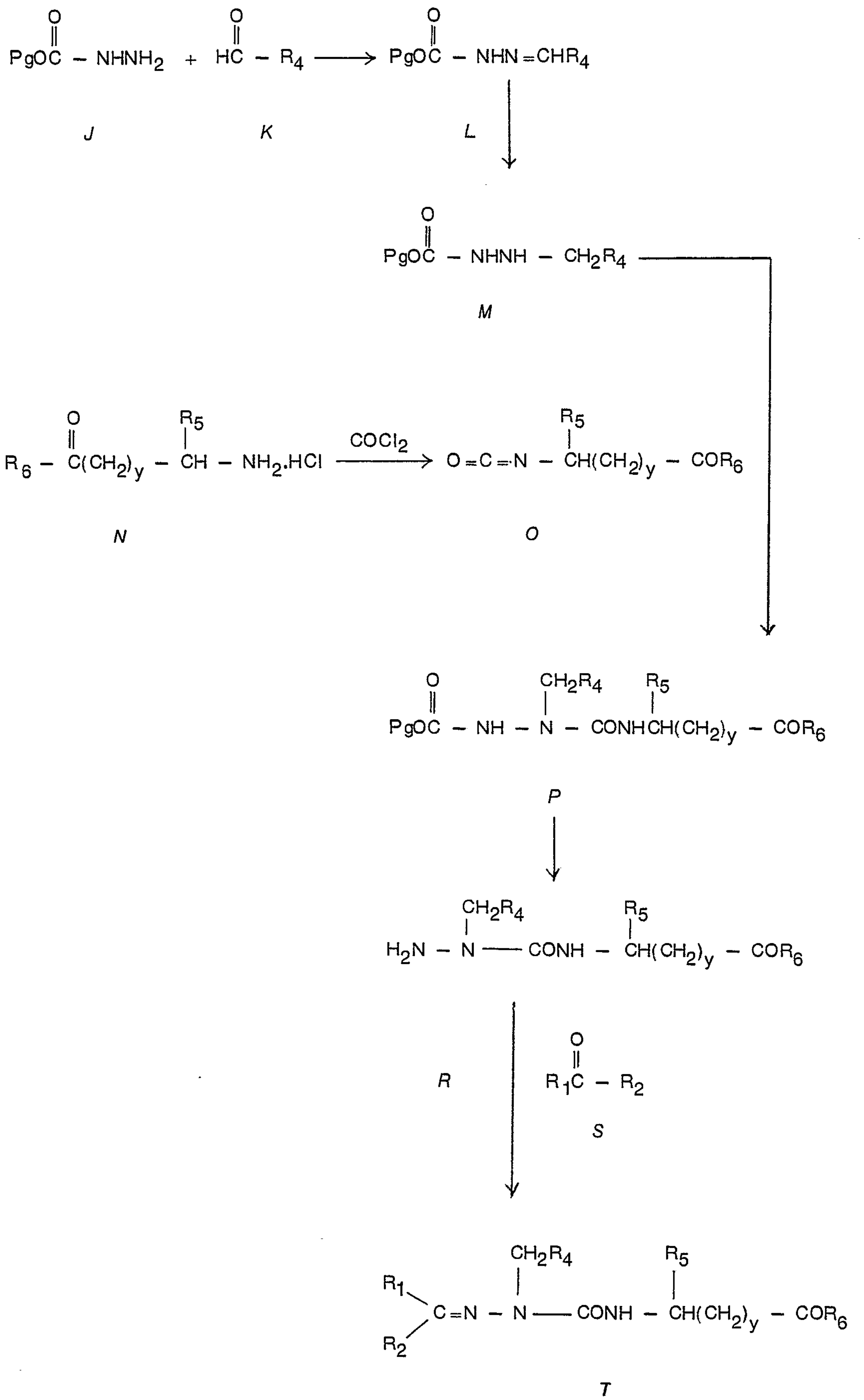


J K L

M

N O

P

R S

T

In the foregoing reaction sequence, an N-protected hydrazine *M* is prepared by condensation of a protected-carboxy substituted hydrazine with an appropriately substituted aldehyde *K*, isolating the resulting compound *L* and then reducing it e.g. by hydrogenation, with hydrogen using a palladium-on-carbon catalyst, or by treating *L* with a reducing agent such as sodium borohydride.

The resulting hydrazine *M* is then reacted with an appropriately substituted isocyanate *O* to form the N-protected amino peptide *P*. Isocyanate *O* can be obtained by treating an α-amino acid ester hydrochloride *N* with phosgene in a hydrocarbon solvent, as illustrated in the above reaction sequence, removing the solvent and distilling the residue.

The protected carboxy group on the terminal amino group of compound *P* can then be removed by any standard method such as by acid hydrolysis using acetic acid or trifluoroacetic acid, to yield the free amine *R* (see for example J. Chem. Soc. Perkin I, 246 (1976)), which is then condensed with an appropriately substituted acid ketone or aldehyde *S* under basic conditions e.g. in the presence of sodium acetate, to yield the hydrazone *T*. For example, using pyruvic acid ethyl ester as ketone *S* yields a hydrazone *T* in which $R_1$ is $—CH_3$ and $R_2$ is $—COOC_2H_5$. $R_6$ in the above sequence may for example, be methoxycarbonyl. The resulting compound *T* can then be reduced e.g. by treatment under weakly acidic conditions with sodium cyanoborohydride, to produce compound *E*.

Alternatively the primary amine *R* can be condensed with ketone or aldehyde *S* in the presence of a reducing agent such as sodium cyanoborohydride under weakly acidic conditions e.g. in the presence of acetic acid, whereby the hydrazone *T* will be reduced, as it is formed, to yield the compound *E* directly.

b) The compounds of the present invention can also be prepared by coupling an amino acid ester having the formula:

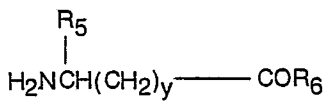

*A'*

with a compound of the formula:

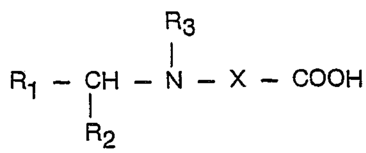

*B'*

in which $R_1$, $R_2$, $R_5$ and $R_6$ may include suitable protection of any reactive groups, followed by removal of any protecting groups, so as to produce a dipeptide of the formula

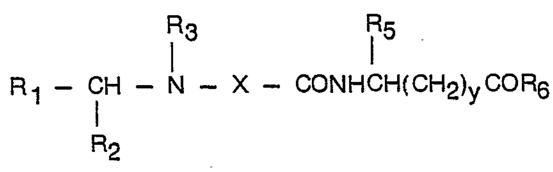

*C'*

and thereafter, if desired, preferably when X is

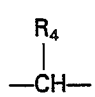 and $R_2$ is other than 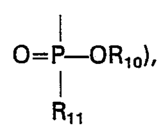),

when $R_6$ is OH, converting $R_6$ into an alkoxy group or the group $—NR_8R_9$ e.g. by the procedure described above.

In the case of the preparation of compounds in which X is

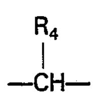 and $R_2$ is other than 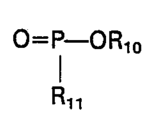

a compound B' can be subjected to reductive condensation an appropriately substituted amino acid with keto acid or ester according to the reaction scheme.

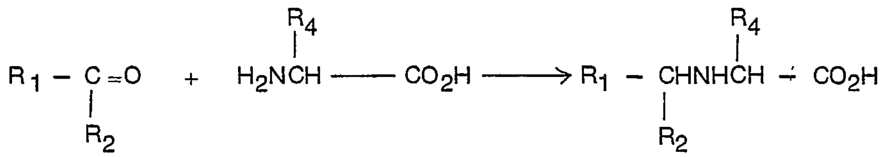

Preferably, however, the compound B′ is prepared by reductive condensation of an appropriately substituted keto acid with an amino acid ester according to the reaction

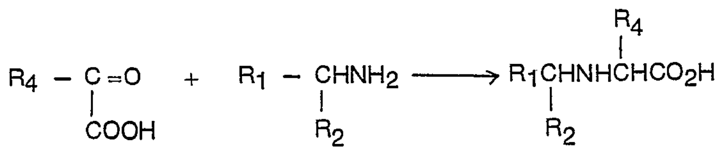

These condensations may be carried out under the conditions described above in connection with the reaction between compounds D and C in method a). The resulting compound B′ is then coupled with an amino acid A′ in which $R_6$ is e.g. alkoxy or dialkylamino, or other non-reactive group within the above definition of $R_6$.

Route b) is suitable for those compounds in which $R_2$ is

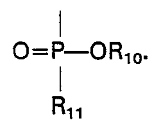

A starting intermediate B′ for such compounds is, e.g. the compound

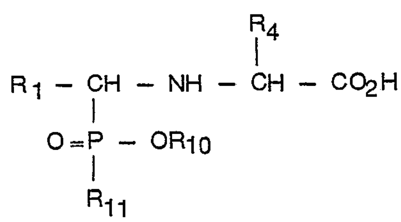

D′

in which $R_{10}$ is alkyl or benzyl. Compound D′ can be prepared for example by reaction of an amine

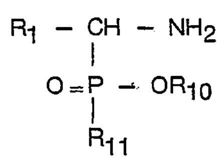

P′

with a substituted pyruvic acid of the formula

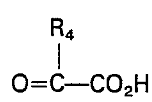

to form a Schiff's base which is reduced, preferably with sodium cyanoborohydride to form compound D′. The compound A′ which is reacted in this particular procedure with compound D′ is for example an appropriately substituted methyl glycinate (i.e. $R_6$ is —OMe), the conditions being e.g. as outlined above in part a) for reactions between primary amines and keto acids or esters, for instance using a condensing agent e.g. DCC.

Appropriate selection of protecting groups as $R_6$ and $R_{10}$ enables the resulting compound selectively to be de-protected at the group $R_6$, by conventional procedures, to provide a terminal carboxyl group in which $R_6$ is —OH, whilst leaving the group $R_{10}$ in position if desired; if desired however, both $R_6$ and —$OR_{10}$ can for example, be hydrolysed to hydroxy under alkaline conditions.

Compound B' can be coupled with an $R_5$ substituted amino acid A' using standard techniques e.g. carrying out the reaction by first forming an activated ester group by reaction of B' with an activating agent such as 1-hydroxybenzotriazole, followed by reaction with compound A' and treatment with a condensing agent such as 1-(3-dimethylaminopropyl)-3-ethylcarbodiimide hydrochloride.

c) A further method for preparing compounds in which X is

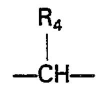

comprises reducing a compound of the formula

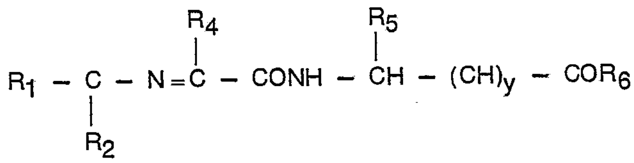

X

in which $R_1$, $R_2$, $R_4$, $R_5$ and $R_6$ may include suitable protection of any reactive groups, so as to produce a compound of the formula

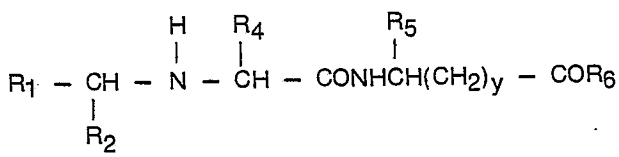

G'

followed by removal of any protecting groups. This method is conveniently carried out by condensation of a ketone of the formula

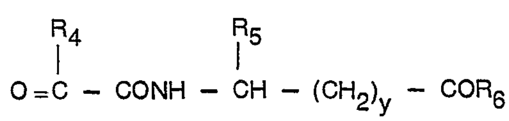

E'

with an amino acid or ester of the formula

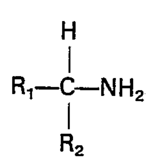

F'

in the presence of a reducing agent e.g. sodium cyanoborohydride.

If desired, the resulting compound G' having a terminal carboxy group ($R_6$=—OH), and preferably those in which $R_2$ is other than

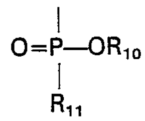

can be further treated by procedures described above to form corresponding compounds of formula I in which $R_6$ is alkoxy or —$NR_8R_9$.

The intermediate E′ in which $R_6$ is —OH can be prepared by following the reaction sequence given below:

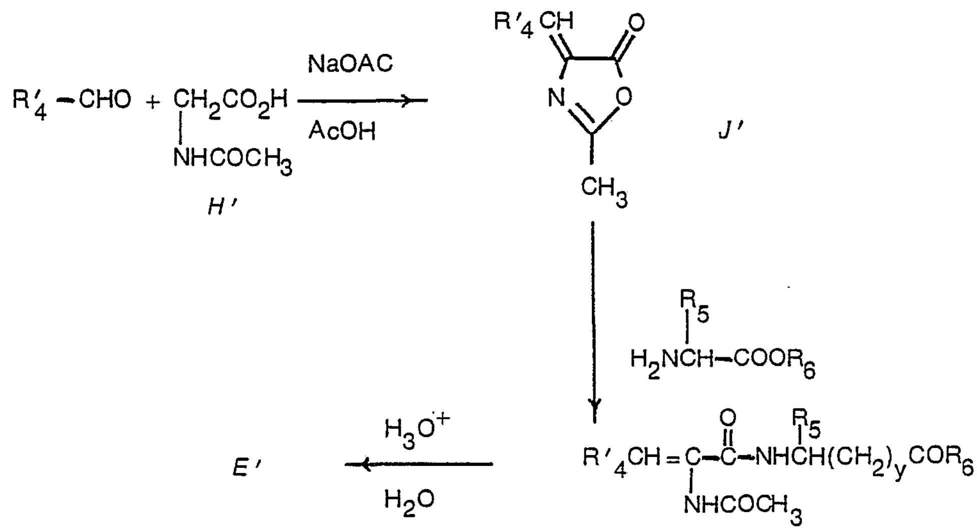


H′ J′ K′

in which $R'_4$ is a group which together with a methyl group by which it is attached to the peptide chain of the resulting compound E′ constitutes an $R_4$ group.

N-Acetyl glycine H′ is condensed with an appropriately $R_4$ substituted aldehyde (preferably an aryl or heteroaryl or heteroaralkyl aldehyde) in the presence of either acetic anhydride, or of acetic acid/sodium acetate, to yield the substituted azlactone J′ which is in turn reacted with an $R_5$ substituted glycinate to yield the $R_5$ substituted N-acetyl dehydro dipeptide which in the presence of aqueous acid is converted to the corresponding $R_4$, $R_5$ substituted pyruvoylamino acid E′.

Alternatively the starting compound E′ may be prepared by condensing an $R_4$-substituted pyruvic acid with an $R_5$-substituted glycinate ester under the influence of dicyclohexyl carbodiimide (DCC) followed by alkaline hydrolysis of the resulting ester according to the reaction sequence:

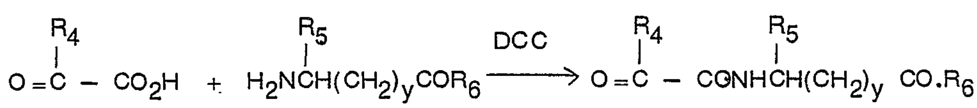


L′ M′ N′

$R_6$ in compound *M′* may for example be methoxy.

Hydrolysis of an ester *N′* gives the free keto acid

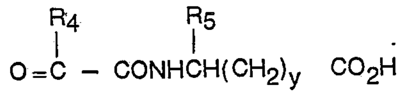

O'

In the case of those compounds in which $R_2$ is

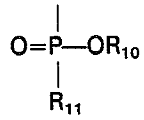

method c) can be practised using compounds in which $R_{10}$ is other than hydrogen i.e. benzyl or lower alkyl. The corresponding compounds in which $R_{10}$ is hydrogen can readily be obtained by reducing $R_{10}$ when it is benzyl, and when it is alkyl by basic hydrolysis, using conventional techniques.

Intermediates which can be used to produce compounds in accordance with the invention by this procedure have the formula:

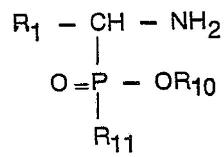

P

in which $R_{10}$ is not hydrogen, and will generally be condensed with a compound of formula E' in the presence of a reducing agent such as sodium cyanoborohydride. The aforesaid intermediates can be prepared for example, by reacting together an $R_1$ substituted aldehyde, ammonia and an $R_{11}$ substituted alkoxy or benzyloxy phosphonic or phosphinic acid.

d) Yet a further method for the preparation of compounds of the invention, comprises reacting an amino acid or ester having the formula

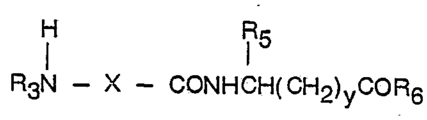

S'

in which X, $R_5$ and $R_6$ are as defined above, with a compound of the formula

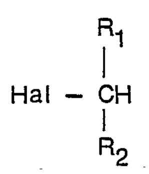

T'

in which $R_1$, $R_2$, $R_4$, $R_5$ and $R_6$ may include suitable protection of any reactive groups, and Hal represents halogen e.g. chlorine or bromine.

If desired, preferably when X is

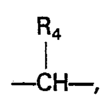

when $R_6$ is (—OH) the $R_6$ group may be converted to an alkoxy group or the group $—NR_8R_9$ by the procedures mentioned above.

The reaction of the alkylating agent T' with intermediate S' can be carried out under conventional alkylating conditions, preferably in the presence of a base, e.g. a tertiary amine on inorganic hydroxide, carbonate or bicarbonate. The reaction is usually carried out in water or an organic solvent such as dimethyl formamide or acetonitrile.

In the case of the compounds in which X is

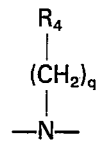

compounds in which $R_3$ is hydrogen may be prepared by following the reaction sequence given below by way of example:

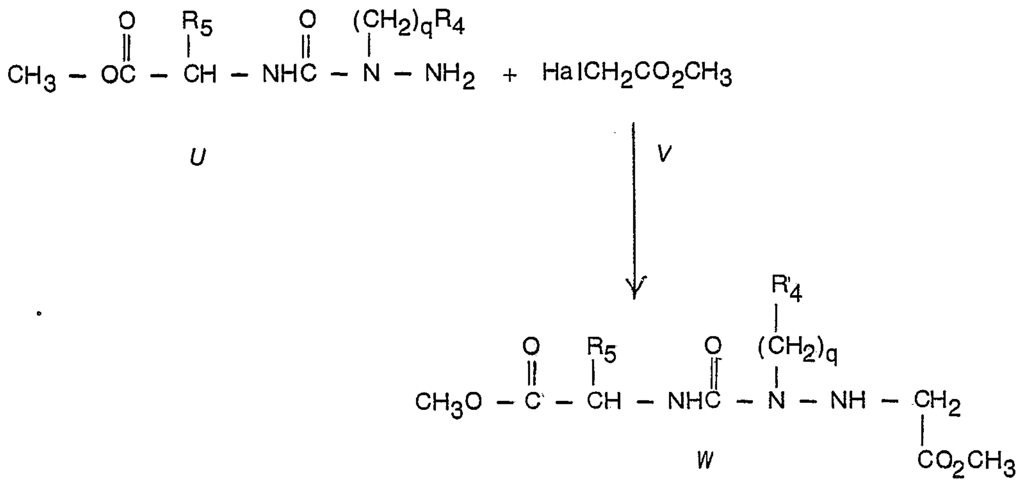

U

V

W

Hydrolysis of the methylated carboxyl groups yields the compounds in which $R_6$ is OH and $R_2$ is —COOH. The halide is preferably chlorine or bromine.

e) Yet a further method is available for preparing those compounds of the inveniton in which $R_2$ is

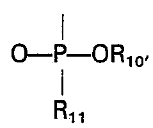

which comprises reacting a phosphite ester or phosphonate ester with an $R_4$, $R_5$ disubstituted compound as shown below:

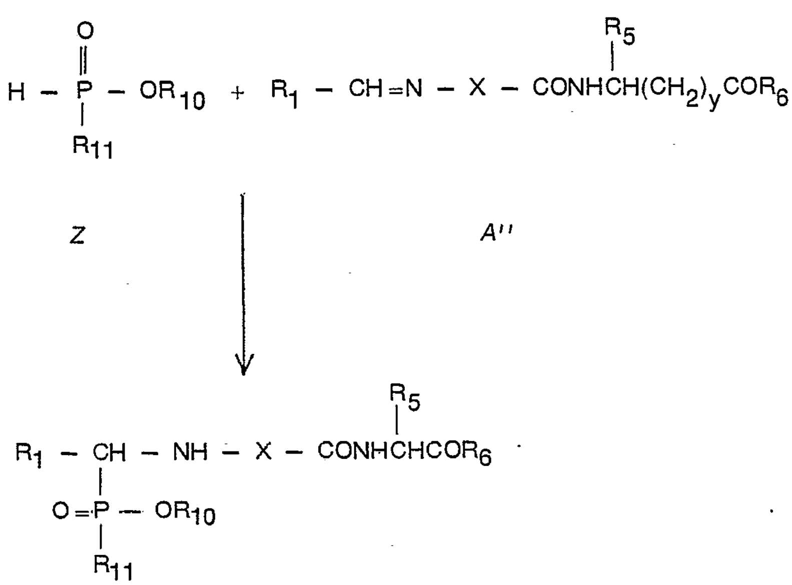

Z

A''

B''

in which $R_{10}$ is not hydrogen, and $COR_6$ is a protected carboxy group e.g. carbomethoxy, and any other reactive groups in $R_1$, $R_4$, and $R_5$ may be suitably protected, followed by removal of the protecting groups as required.

The resulting compound B'' may be treated to deprotect the group $COR_6$ so as to provide a terminal carboxylic group ($R_6$ = OH). Also the group $R_{10}$ may if desired be replaced by hydrogen. For example, treatment of compound B'' in which $R_6$ is lower alkyloxy e.g. methoxy, by hydrolysis, under basic conditions yields compounds of the invention in which $R_{10}$ is hydrogen and $R_6$ is —OH.

The immediately preceding method is particularly suitable for those compounds in which X is

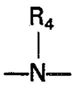 and also those in which X is 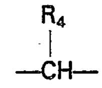

and $R_4$ is hydrogen, aryl, heteroaryl, adamantyl or indolyl directly attached to the carbon of the peptide chain.

The present invention comprehends the salts of those compounds of formula I containing one or more free acid or basic groups, with inorganic or organic acids or bases. Such salts include, for example, alkali metal salts e.g. sodium and potassium salts and salts of organic and inorganic acids, for example, HCl and maleic acid.

The salts may be formed by known methods, for example, by reacting the free acid or free base forms of the product with at least one equivalent of the appropriate base or acid in a solvent or reaction medium e.g. ethylacetate, in which the salt is insoluble, or in a solvent which can be removed in vacuo or by freeze-drying.

When the reactions described above, either for the preparation of intermediates or the final desired compounds of the invention, involve the generation of water, e.g. condensation reactions, these reactions may be carried out under azeotropic distillation with a suitable high boiling solvent e.g. toluene or xylene, or in the presence of a dehydrating agent e.g. a molecular sieve.

Various intermediates for the preparation of the compounds of the invention by the methods described above are commercially available e.g. from Chemical Dynamics Corporation or their preparation is also described in the peptide or general chemical literature e.g. J. H. Jones Comprehensive Organic Chemistry, Vol. 2. p. 819—823 1979; references 2 and 29—31, and Tetrahedron Lettters No. 4, 1978, p. 375—378.

The compounds defined above may possess centres of chirality at their asymmetric carbon atoms.

Generally, the compounds produced by the processes described herein will be diastereomeric mixtures of compounds which are respectively 'D' and 'L' at least at one of the said centres of chirality.

Separation of such diastereomeric mixtures may be carried out by techniques well known in the art. For example, the respective compounds can be separated by physical means such as crystallization or chromatography, or by the formation of diastereomeric salts followed by fractional crystallisation.

The invention is concerned with all stereomeric forms of the dipeptide compounds set forth herein.

Those compounds having a configuration similar to that of natural L-amino acids are preferred. Usually natural amino acids are assigned the *S* configuration according to the Cuhn-Ingold Prelog system. A notable exception is the natural amino acid Cysteine which has the R configuration.

Dipeptide compounds as defined herein inhibit the activity of enkephalinases. Some of these compounds have been found useful inhibiting Enkephalinase A derived from the striata of rats. In *in vitro* tests, such compounds, when used in a concentration range of $10^{-9}$ to $10^{-6}$M, have reduced the activity of the aforesaid enzyme by 50% or more.

The enkephalinase used in the aforesaid tests was obtained by separation from the brains of young male Sprague-Dowley rats using the procedure described by C. Gorenstein et. al. in Life Sciences, vol., 25, Pages 2065—2070, Pergamon Press (1979).

In this procedure the various enkephalin degrading enzyme activities in brain are resolved from one another following a procedure described by Gorenstein and Synder (Life Sciences, Sciences, Vol. 25, pp 2065—2070, 1979). The brain (minus cerebellum) from one young Sprague-Dawley rat was first homogenised in 30 volumes of 50 mM Tris buffer, pH 7.4. The resulting homogenate was centrifuged at 50,000 xg for 15 mins and the pellet, constituting the membrane-bound enzyme material, resuspended in Tris and washed and centrifuged as described above four times.

The membrane pellet was solubilized by incubating it for 45 mins at 37°C. in the presence of 15 volumes (base on initial brain weight) of 50 mM Tris — 1% TRITON X-100 buffer pH 7.4. After centrifugation at 100,000 xg for 60 mins to remove non-solubilized material, the Triton-soluble supernatant was layered on a 1.5 × 30 cm DEAE Sephacel column previously equilibrated with 50 mM Tris — 0.1% Triton, pH 7.4. Material was eluted from the column using a 1 litre linear NaCl gradient running from 0.0 to 0.4 M. Eluant was collected in 7 ml fractions, each of which was assayed for enkephalinase activity. Under these conditions enkephalinase "A" activity (dipeptidyl carboxypeptidase) is found to elute between 120 and 220 ml, followed by aminopeptidase activity (260 to 400 ml) and finally enkephalinase "B" activity (dipeptidyl aminopeptidase) between 420 and 450 ml.

In the array enkephalinase activity was monitored by radiometry. The substrate was 3H-met-

enkephalin (50.1 Ci/mmol, NEN) diluted in 0.05 M Tris buffer, pH 7.4, such that the final reaction mixture concentration was 40 nM. Total reaction mixture volume including enzyme and substrate was 250 ml.

Incubation was carried out for 90 min at 37°C. To kill the reaction, tubes were transferred to a boiling water bath for 15 min. A 4 ml aliquot of the reaction mixture was then spotted on a Baker-Flex Silica Gel 1B plate (20 × 20 cm) along with unlabelled-standards of met-enkephalin, tyrosine, tyrosyl-glycine, tyrosyl-glycyl-glycine) and the components co-chromatographed in an isopropanol: ethyl acetate: 5% wt/vol acetic acid solvent system (2:2:1) which is capable of resolving met-enkephalin from its breakdown products. Total running time was approximately 17 hours. TLCtanks were gassed with nitrogen prior to starting the run. Following the run, the markers were visualized with ninhydrin spray. These spots along with remaining plate regions were cut from the plate and the radioactivity corresponding to each spot monitored through scintillation counting. Thus the amount of radioactivity in the spots containing respectively Tyr, TyrGly, TyrGlyGly, and undigested metenkephalin, was determined.

For each plate, the radioactivity of each spot produced therefrom can be expressed as a percentage of the total radioactivity recovered from the plate.

To eliminate the effect of natural decomposition of metenkephalin in the absence of enzymes, the percentages of the radioactivity of the Tyr, TyrGly, TyrGlyGly spots of a non-enzyme blank are subtracted from the corresponding values obtained for the experimental samples from each of the runs using enzymes; the resulting values are summed to give net product percentages for each run.

In order to obtain figures for making comparative evaluations of different dipeptide species, for each species the net product percentage (P) formed in a run in the presence of a respective species and the net product percentage (A) formed in a test run in the absence of the species can be substituted into the formula (A—P)/P. The resulting values for different species are used to provide an indication of the comparative inhibiting activity of the various dipeptides.

Also, in *in vitro* tests, such compounds, when parenterally administered in the dose range of 5 to 100 mg/kg to mice, have been observed to potentiate the analgesic effects of intracerebrally administered D-Ala-met-enkephalinamide.

Pharmaceutical compositions may be in any of the forms known in the art, for example, tablets, capsules or elixirs for oral administration, and sterile solutions or suspensions for parenteral administration.

The dosage forms are advantageously prepared using, in addition to the active dipeptide, a pharmaceutically acceptable and compatible carrier or excipient, binders, preservatives, stabilizers and flavouring agents.

The dosage forms will usually be prepared such as to facilitate the administration of the active compounds in a dosage in the range of from 5 to 100 mg/kg, such doses being administered at intervals of 3 to 8 hours.

Typical acceptable pharmaceutical carriers for use in the formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as di-calcium phosphate and tri-calcium phosphate; sodium sulfate, calcium sulfate, polyvinylpyrrolidone, polyvinyl alcohol, stearic acid; alkaline earth metal stearates such as magnesium stearate and calcium stearate, stearic acid, vegetable oils such as peanut oil, cotton seed oil, sesame oil, olive oil and corn oil; cationic and anionic surfactants; ethylene glycol polymers; betacyclodextrin, fatty alcohols and hydrolysed cereal solids.

The following Examples illustrate the invention

## Example 1

### Preparation of L-3-(3′-thienyl)alanyl-L-alanine trifluoroacetate salt.

a) N-tert-butyloxycarbonyl-L-3-(3′-thienyl)alanine.

2.0 g (11.68 mM) of L-3-(3′-thienyl)alanine (Chemical Dynamics Inc.) were suspended in 1.6 ml triethylamine, and 2.6 g of di-tert-butyl dicarbonate were dissolved in a mixture of 22 ml of dimethylformamide (DMF) and 22 ml of methanol which had previously been dried over a molecular sieve.

The DMF/methanol solution was added to the suspension with stirring and stirring was continued over a further period of 24 hours at ambient temperature. The resulting, homogeneous solution was diluted with 330 ml of water and then acidified with citric acid. The resulting solution was extracted 4 times with 80 ml of ethyl acetate, the extract was washed with water, then with brine, and dried over magnesium sulphate. The solvent was evaporated to give 3.2 g of the title product.

b) N-tert-butyloxycarbonyl-L-3-(3′-thienyl)alanyl-L-alanine ethyl ester.

The 3.2 g (11.68 mM) of title product from step a) were suspended, together with 1.8 g of L-alanine ethyl ester hydrochloride (Sigma Chemicals) and 3.3 g hydroxybenzyltriazole in a mixture of 20 ml dimethylformamide and 2.0 ml of triethylamine with stirring at ambient temperature. 2.5 g of N-(N,N-dimethylaminopropyl)-N′-ethylcarbodiimide hydrochloride and 2 ml of triethylamine were then added to the suspension and the resulting mixture was stirred overnight. 300 ml of water wsa then added to the reaction mixture which was thereafter extracted 4 times with 60 ml ethyl acetate. The combined extracts were washed twice with 70 ml of 1N sodium bicarbonate, twice with 30 ml of 1N citric acid, twice with 70 ml

of water, and once with 70 ml brine. The resulting extract was dried over magnesium sulphate and the solvent evaporated to give 5.1 g of the title product.

c) N-tert-butyloxycarbonyl-L-3-(3′-thienyl)alanyl-L-alanine

4.4 g(11.8 mM) of the title product from step b) was dissolved in 30 ml of methanol. 30 ml water was added to the solution followed by 1.1 g (14.2 mM) of NaOH in the form of a 50% wt by volume aqueous solution. The reaction mixture was stirred at ambient temperature and after one hour a sample was checked for starting material using thin layer chromatography (tlc) on silica gel using a mixture of methanol, chloroform and acetic acid in a volumetric ratio of 5:95:0:5 as the mobile phase. Stirring was continued and the reaction mixture monitored in this way until completion of the reaction was indicated by the absence of starting material. The reaction mixture was then concentrated under reduced pressure and the residue was mixed with 30 ml water; the resulting aqueous solution was washed twice with 30 ml of methylene chloride and then acidified to yield a gummy precipitate. This precipitate was extracted with ethyl acetate and the extract dried over magnesium sulfate after which the ethyl acetate was evaporated to give 5.8 g of the title product.

d) L-3-(3′-thienyl)alanyl-L-alanine trifluoroacetate.

The 5.8 g of the title product from step c) was dissolved in 15 ml methylene chloride with stirring. 15 ml trifluoroacetic acid was added to the solution and the mixture was stirred for two hours at room temperature. The reaction mixture was monitored by tlc on silica gel using a mixture of methylene chloride, methanol and acetic acid in a volumetric ratio of 95:5:0:5 as the mobile phase. When tlc indicated the absence of starting material the reaction mixture was concentrated to a gummy residue which was dissolved in benzene and distilled to a residue, dissolved again in benzene and distilled yet again to give 7 g of the title product.

e) N-(1-carboxy-3-phenylpropyl)-L-3-(3′-thienyl)alanyl-L-alanine

A solution of L-3-(3-thienyl)alanyl-L-alanine trifluoroacetate (4.75 g, 13.4 mmol) and 2-oxo-4-phenylbutyric acid (11.0 g, 56.1 mmols) in water was adjusted to pH 6.8 by addition of 1N NaOH. Sodium cyanoborohydride (2.2 g, 34.8 m,mol) was added in a single portion to the solution and the resulting reaction mixture was stirred at room temperature for 64 hours. The pH of the resulting solution was then adjusted to 2.9 by the addition of 1N HCl. The gummy product which separated from the solution was washed several times with water. The gummy product was then dissolved in 20 ml of acetonitrile and the solution diluted with 10 ml of ether. Upon cooling a solid separated and this was collected by filtration. The mother liquors were stood in a refrigerator for two days by which time a white solid had deposited: this solid was filtered off, washed with acetonitrile and dried to yield the N-(L-1) diastereoisomer of the title compound having a m.p. of 192.5°C—193°C.

The first mentioned solid was recrystallized from ethanol to give the N-(D-1) diastereoisomer having a m.p. of 163°C.—164°C.

## Examples 2 to 7

By following procedures similar to that of Example 1, using appropriate starting materials, the following compounds and diastereomeric mixtures were prepared:

2) N-(L-1-carboxy-3-phenylpropyl)-L-phenylalanyl-l-alanine, m.p. 176°C.—179°C;
3) N-(D,L-1-carboxy-3-phenylpropyl)-L-3-(2′-naphthyl)-alanyl-L-alanine, m.p. 149°C.—155°C.
4) N-(L-1-ethoxycarbonyl-3-phenylpropyl)-L-3-(2′-thienyl)alanyl-L-alanine maleate, m.p. 124°C.—125°C.
5) N-(D,L-1-carboxyl-ethyl)-L-phenylalanyl-L-alanine, m.p. 125°C.—135°C.
6) N-(D-1-carboxy-3-phenylpropyl)-L-(4-fluorophenyl)-alanyl-L-alanine, m.p. 147°C.—152°C.
7) N-(D,L-1-carboxy-3-phenylpropyl)-L-phenyl-alanyl-glycine, m.p. 140°C.—145°C.

## Example 8

15 g (0.07 m) of phenylalanine methyl ester hydrochloride were dissolved in 400 ml methanol. 2.4 g (0.105 m) of sodium phenyl pyruvate, followed by 15 g powdered 3A molecular seive (Aldrich Chemical) were then added. The slurry was stirred at ambient temperature while 6.6 g (0.105 m) of sodium cyanoborohydride in 75 ml methanol were added over a period of 6 hours. The slurry was thereafter stirred for 2 days, then filtered and the filtrate concentrated to a syrup. 300 ml of 2 1/2 per. cent. hydrochloric acid were added to the syrup over 3 hours and the resulting white crystals filtered and dried under reduced pressure to constant weight. A representative sample of the dried product was chromatographed on silica gel thin layer plates using as solvent system a chloroform: methanol: concentrated hydrochloric acid mixture (50:15:1 volumetric ratio) to ascertain the approximate ratio of the diastereoisomers. The chromatogram showed approximately a 50/50 mixture of diastereoisomers with some 3-phenyl-2-hydroxy porpionic acid present.

Yield: 24 g; m.p. 119°—130°C.

NMR consistent with the desired product.

Upon crystallisation from 400 ml of ethanol 14.7 g of desired compound (again in the form of a

diastereomeric mixture) was obtained enriched with the faster moving isomer (as determined by thin layer chromatography (tlc), M.P. 135°—150°C.

200 mg of the enriched diastereomeric mixture was recrystallized from 3 ml of methanol to yield 100 mg crystals which were nearly pure faster moving isomer (as determined by tlc) M.P. 169°C—172°C.

14.5 g of the enriched diastereomeric mixture was subjected to a recrystallisation procedure using 400 ml methanol but no crystals formed. The solution was concentrated to 200 ml, seeded and allowed to stand. A gelatinous precepitate formed which was shown by tlc to be a mixture of isomers with the slower moving isomer predominant: yield 7.0 g, 3.0 g of this product were subjected to high performance liquid chromatography (hplc) on Waters Prep. 500 Equipment using two silica gel columns in series, with a solvent system consisting of chloroform: methanol: ammonium hydroxide (500:150:10 volumetric ratio) at a flow rate of 0.2 litres per minute. Fractions 8 and 9 yielded 450 mg of isomer I (see below): Fraction 10 yielded 400 mg of material which upon recrystallisation from methanol yielded 150 mg of isomer I. Fractions 12 to 15 yielded 1.2 g pure isomer II (see below). Fraction II yielded a mixture of isomer I and isomer II.

HPLC was carried out on another 3.5 g of the same product as in the preceding paragraph using the same solvent ratios and absorbents. Fraction 4 yielded 400 mg of pure isomer I, Fraction 5 yielded 1.0 g of material which when recrystallised from methanol yielded 550 mg of substantially pure isomer I, fraction 6 yielded 0.4 g of mixture of isomer I and isomer II, fractions 7 to 10 yielded 1.6 g of pure isomer II.

Isomer I M.P. 171°—173°C.
Mass Spectrum M + I
328
282 (M—COOH)
268 ($M—COOH_3$)
236 (M-91)
Formula

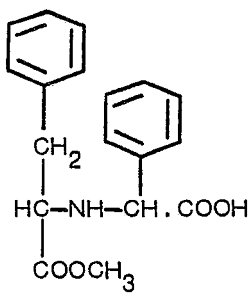

Isomer II M.P. 149°—151°C.
Mass Spectrum M + I
328
282 (M—COOH)
268 ($M—COOCH_3$)

The filtrate obtained upon filtering the gelatinous precipitate was concentrated to 50 ml and 100 ml of acetonitrile were added thereto with cooling, resulting in precipitation of 1.0 g of white crystals M.P. 167°—170°C. These crystals were recrystallised from methanol; 0.7 g of white crystals, M.P. 171°—173°C were obtained.

L-N-[(1-carboxy-2-phenyl)ethyl]-phenylalanyl-β-alinine methyl ester

1.0 g (3.05 mM) of isomer I were dissolved in 20 ml of dimethylformamide (dmf) then were added 520 mg (3.4 mM) of 1-hydroxy-benzotriazolehydrate (HOBT $H_2O$) followed by by 472 mg (3.4 mM) of β-alanine methyl ester hydrochloride, 1.3 ml (equivalents) of N-ethylmorpholine (NEM) and 649 mg (3.4 mM) of N-(3-dimethylaminopropyl)-N-ethylcarbodiimide hydrochloride (EDCL). The resulting solution was stirred at room temperature for 48 hours and then poured into 150 ml of ice water with stirring and the aqueous solution extracted twice with 150 ml of ethylether. The ethylene layer was washed three times with 100 ml of water, then with water to which a few drops of HCL had been added, and then finally with water. The ether layer was dried over magnesium carbonate, filtered and the filtrate concentrated to a gum; Yield 1.1 g of title product.

L-N-[(1-carboxy-2-phenyl)ethyl]-phenylalanyl-β-alanine

The gum from the previous step was dissolved in 20 ml methanol and 6.6 ml of 1N sodium hydroxide added dropwise over a period of 10 minutes with stirring and external cooling. The solution was allowed to stand overnight (15—20 hours) at room temperature. The 5 per. cent. hydrochloric acid solution was added dropwise until the pH of the solution reached 2.5 (the solution being stirring during this addition) whereupon a white solid slowly precipitated. The solution was filtered, and the precipitate dried under reduced pressure, at 40°C., to constant weight.

Yield 900 mg. M.P. 218°—219°C.

Mass Spectrum M/e

385 (M + I)

367 (M + I-18)

340 (M + I-COOH)

339 (M-45-COOH)

293 (M-91).

Examples 9 to 15

By following procedures similar to that of Example 8 using appropriate starting materials, the following compounds and diastereomeric mixtures were prepared.

9) N-(L-1-carboxy-3-phenylpropyl)-D,L-3-(1-naphthyl)alanyl-L-alanine m.p. 186°C.—194°C.

10) N-(L-1-carboxy-3-phenylpropyl)-L-3-(1-naphthyl)alanyl-β-alanine.

11) N-(L-1-carboxy-2-methylpropyl)-D,L-phenyl-alanyl-L-alanine m.p. 160°C.—172°C.

12) N-(L-1-carboxy-2-(benzylthio)-ethyl)-L-phenylalanyl-β-alanine m.p. 152°C—154°C.

13) N-(L-1-carboxy-3-phenylpropyl)-L-phenylalanyl-L-alanine.

14) N-(L-1-carboxy-2-phenylethyl)-L-phenylalanyl-δ-amino butyric acid m.p. 209°C.—211°C.

15) N-(L-1-carboxy-3-phenylpropyl)-L-phenyl-alanyl-β-alanine hemihydrate m.p. 176°C.—190°C.

Example 16

N-(D,L-1-carboxyethyl)-L-phenylalanylglycine.

To a solution of 0.6 g (2.5 m. mol) of L-phenylalanylglycine in 5 ml of 1N NAOH and 20 ml water is added dropwise over a period of 1 hour a solution of 0.25 g (2.5 m. mol) 2-chloropropionic acid in 5 ml of 1N NaOH; during the addition the reaction solution is maintained in the temperature range of from 80°C.—90°C. and the pH thereof in the range of 8—9. On completion of the addition the reaction mixture is maintained, by heating, for 15 minutes at 80°C.—90°C. and then evaporated to dryness under reduced pressure and neutralised with 5% wt./vol. aqueous hydrochloric acid. Upon re-evaporation to dryness a grey solid is obtained.

The grey solid is stirred with ether which is then decanted; and the solids are then stirred with cold ethanol. Undissolved solids are filtered from the ethanol solution which is evaporated to dryness to give the title mixture of diastereomers. m.p. 90°C.—120°C.

Example 17

a) Preparation of t-butyloxycarbonyl hydrazine

To a stirred solution of 0.1 ml hydrazine hydrate in 15 ml isopropanol was added dropwise a solution of di-t-butyl dicarbonate in isopropanol (the solution having a concentration of 0.45 ml of dicarbonate in 7 ml of the propanol). The addition was at the rate of one drop every 7 seconds. The reaction was stopped after approximately 6/7ths of the t-butyldicarbonate had been added.

The mother liquor was evaporated, and the resulting solid was chromatographed on silica gel using a mixture of chloroform/methanol in a volume ratio of 97:3 as the mobile phase.

Fractions 20 to 33 were collected which after working up in conventional manner yielded 0.105 g of the title product as a solid m.p. 34°C.—35°C.

Re-crystallisation from ether/petroleum ether yielded a solid with m.p. 36.5°C.—37°C.

b) Preparation of Benzaldehyde hydrazino-t-butylcarboxylate

3.47 g of the title product from a) were dissolved in 30 ml of ethanol with stirring and 2.79 g benzaldehyde were added. The reaction mixture was heated in a steam bath with stirring for 10 minutes then chilled in an ice water bath. The resulting reaction mixture was filtered to yield the title product (3.05 g).

The mother liquor was evaporated to a solid which after washing with hexane and filtering yielded 1.56 g solids.

The two fractions were subjected to thin layer chromatography on silica gel plates using chloroform/methanol in a volume ratio of 97:3 as the mobile phase.

Subsequent tests revealed that the two fractions were substantially identidal products. The total yield was 5.06 g.m.p. 190°C.—194°C.

c) Preparation of benzylhydrazino-t-butylcarboxylate

1 g of the title product from b) was dissolved in 30 ml tetrahydrofuran. 0.23 g of a 5% palladium-on-carbon catalyst was added and hydrogenation was carried out for approximately 15 minutes. The reaction mixture was filtered and the mother liquor evaporated yielding 0.96 g of the title product.

d) Preparation of methyl-1-isocyanato-3-methylpropionate

7 g of L-leucine methyl ester was suspended in 200 ml toluene which had previously been dried over calcium hydride. The suspension was stirred and an excess of phosgene gas bubbled therethrough. After

the solids had dissolved the temperature of the reaction mixture was raised to, and maintained at, reflux for a period of half an hour.

After cessation of heating nitrogen gas was bubbled through the resulting solution to remove the excess phosgene and hydrogen chloride reaction product dissolved in the solution. After this operation the solution was evaporated and the resulting liquid product distilled.

$[\alpha]_D^{26} = -25.55°$. (C = 5.7% in toluene) (literature −22.4°C. Annalen 1952, 575, 217)

b.p. 71°C.—72°C. (45 mm Hg)

e) N-t-butyloxycarbonyl-L-α-azaphenylalanyl-L-leucine methyl ester

2.47 g of benzylhydrazino-t-butylcarboxylate (product of step c) was dissolved in 15 ml toluene with stirring. Then 1.09 g of methyl-1-isocyanate-3-methylpropionate (product of step d) was added, followed by the addition of 1.12 g of triethylamine, to the reaction solution. The resulting reaction mixture was stirred for 16 hours, after which it was diluted with 200 ml of ethyl acetate, washed once with 5% wt./vol. aqueous solution of citric acid, then once with brine, and finally once with water.

The solution was dried over sodium sulphate, and, after filtering off the sodium sulphate, was concentrated to a residue which was chromatographed on 75 g of silica gel using a 7/3 by volume mixture of hexane and chloroform as the eluant.

After working up in conventional manner 3.72 g of the title product was obtained. $[\alpha]_D^{26} = -7.1°$ (C = 3.29% in chloroform).

f) α-azaphenylalanyl-L-leucine methylester

8.0 ml of an anhydrus mixture of $HCl/CH_3COOH$ (volumetric ratio 1/80) was cooled to 4°C. under stirring and the product of step e) was added thereto. Stirring was continued at 4°C. for 20 minutes after which the solvent was removed under reduced pressure. The residue was subjected to thin layer chromatography on silica gel using a chloroform/methanol mixture (98/2 v/v) as the eluant. Appropriate fractions with corresponding rf values were combined yielding 0.163 g of the title product $[\alpha]_D^{26} = +1.9°$.

Mass spectrum

M + 293 (—HCl)

262 (-m-$OCH_3$)

234 (-m-$CO_2CH_3$)

g) N-(D,L-1-ethoxycarbonyl-2-phenyl-ethyl)-α-azaphenyl alanyl-L-leucine methyl ester

0.151 g (0.458 m. mol) of the title product from step f) was dissolved in 3 ml methanol and 83.4 mg of ethyl-4-phenyl-2-oxobutanoate was added thereto with stirring at 60°C. The reaction was monitored by tlc using silica gel plates and a chloro/form/acetone mixture (9/1 by volume) as the eluant. Upon substantial completion of the reaction 70 mg of sodium cyanoborohydride and 4 drops of a 10% wt/vol aqueous solution of acetic acid were added.

The reaction was monitored by tlc using silica gel plates and a mixture of chloroform and methanol (98/2, by volume) as the eluant. A further 70 mg of sodium cyanoborohydride and 4 drops of acetic acid solution mentioned above were then added. The reaction solution was allowed to stand for 16 hours, after which the solvent was removed under reduced pressure. 40 ml ethylacetate was added to the residue, the resulting solution washed with 20 ml of aqueous sodium chloride and then twice with 20 ml water. The organic solution was dried over magnesium sulphate and filtered and the solvent removed under reduced pressure.

The residue (0.277 g) was chromatographed on silic a gel using a mixture of hexane/chloroform (7/3 by volume) as the eluant. Appropriate fractions were combined according to their thin layer migration to give the title product.

h) N-(D,L-1-carboxy-2-phenyl-ethyl)-α-azaphenylalanyl-L-leucine

0.428 g (0.265 m. mol) of the title compound of step g) was dissolved in 10 ml aqueous acetone (acetone/water, 3/1 by volume) 1.06 ml of aqueous sodium hydroxide solution (containing 1.06 m. mol NAOH) was added to the reaction solution whilst stirring. The reaction was monitored using tlc with a chloroform methanol/ammonium hydroxide in a 1:1:1 volumetric ratio as the eluant. An additional 0.04 ml of concentrated sodium hydroxide (containing 0.4 m. mol NAOH) solution was added and the monitoring of the reaction continued until the reaction was substantially complete.

10 ml of water was added to the reaction mixture which was extracted twice with 30 ml portions of ethyl acetate. The extracts were combined, dried and concentrated to a residue.

The residue was chromatographed on silica gel using the lower phase of chloroform: methanol: ammonium hydroxide mixture (2:1:1 volumetric ratio) as the eluant.

Appropriate fractions were combined according to their rf values. The combined fractions were recrystallised from diethyl ether, the mother liquor from the first recrystallisation being further concentrated to provide a second crop of crystals. The total yield of title compound was 43 mg.

Example 18

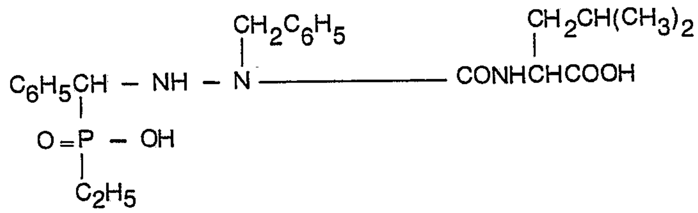

A solution of α-azaphenylalanyl-L-leucine methyl ester (see Example 17) and one equivalent (with respect to the ester) of benzaldehyde in anhydrous methanol is allowed to stand over a 3A type molecular sieve for 16 hours. To the resulting solution is added one equivalent (with respect to the ester) of ethyl phosphonic acid ethyl ester and a trace of sodium methoxide. The reaction solution is then allowed to stand for 6 to 8 hours. The molecular sieve material is then removed by filtration and the filtrate is concentrated under reduced pressure. To the concentrated solution is added 2.1 equivalents (with respect to the ester) of sodium hydroxide in the form of a 1N aqueous solution, and the mixture is stirred for 6 hours at room temperature. The organic solvent is removed under reduced pressure and the residue is diluted with water. Insoluble material is extracted with ethyl acetate and the aqueous solution is acidified to pH 2.5 by the addition of 1N HCl. The resulting precipitate is filtered off and dried to give a mixture of diastereomers of the title compound.

Example 19

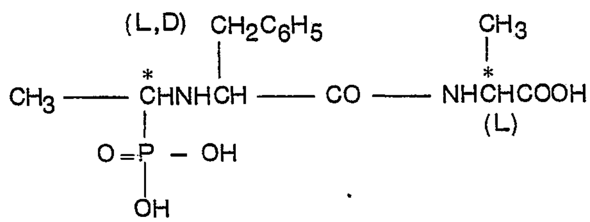

(1L)-1-amino ethyl phosphonic acid (see U.S. Patent Specification 4016148) is dissolved in anhydrous methanol and an equimolar quantity of thionyl chloride is added thereto a drop at a time, the solution being kept at 0°C. during the addition. After the addition is completed the resulting solution is allowed to warm to room temperature and stood at this temperature for from 6 to 8 hours. Volatiles are then removed under reduced pressure at room temperature to yield crude (1-L)-1-aminoethyl phosphonic dimethyl ester hydrochloride.

A solution is made up of the dimethyl ester and an equimolar quantity of sodium phenyl pyruvate in methanol and is adjusted to pH in the range of 6.5 to 7 with 1N NaOH solution and treated with a 50% molar excess of sodium cyanoborohydride. The reaction mixture is stirred for 2 days at room temperature, then poured into water. The pH of the resulting aqueous mixture is then adjusted to a value in the range of 2 to 3, and the resulting precipitate extracted with ethyl acetate. The extracts are washed with water and dried. Evaporation of the solvent gives the crude mono-acid of formula:

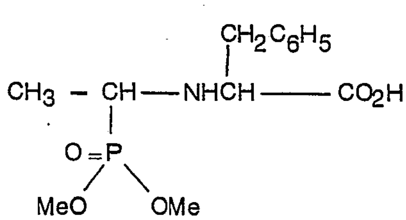

The mono-acid is dissolved in dimethylformamide together with an equivalent (with respect to the mono-acid) of L-alanine methyl ester hydrochloride, and one equivalent (with respect to the mono-acid) of N-ethylmorpholine. The resulting mixture is cooled to approximately 0°C. and one equivalent (with respect to the mono-acid) of N-(3-dimethylaminopropyl)-N′-ethylcarbodiimide hydrochloride is added thereto.

The reaction mixture is allowed to warm to room temperature and stirred overnight. It is then poured into water and the precipitated product extracted with ethyl acetate. The extracts are washed with 0.5% wt/vol HCl, then with saturated sodium bicarbonate solution, dried and evaporated to yield a compound of the formula:

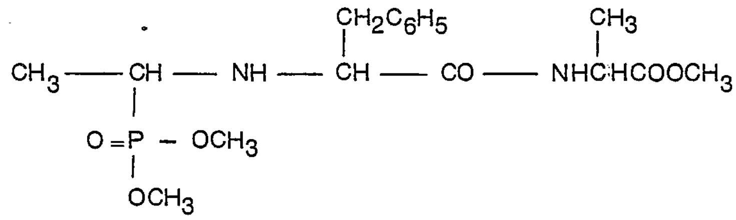

This product is dissolved in methanol, and to the resulting solution is added, drop by drop, 3.1 equivalents with respect thereto of sodium hydroxide in the form of a 1N solution. After stirring overnight at room temperature the reaction mixture is concentrated under reduced pressure, diluted with water and adjusted to pH in the range 3 to 4 with 1N HCl solution.

The precipitated products are filtered, washed with water and dried to give a mixture of diastereomers of the title compound.

**Claims for the Contracting States: BE CH DE FR GB IT LI NL SE**

1. A compound having the formula:

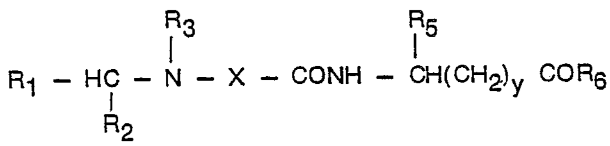

I

and the pharmaceutically acceptable salts thereof, in which

$R_1$ is hydrogen; alkyl; halogen loweralkyl; hydroxy loweralkyl; loweralkoxy loweralkyl; aryloxy loweralkyl; amino loweralkyl; loweralkylamino loweralkyl; diloweralkyl aminoloweralkyl; acylamino loweralkyl; diarylaminoloweralkyl; arylamino loweralkyl; guanidino loweralkyl; heteroaryl; aryl, aralkyl; mercapto loweralkyl; arylthio loweralkyl; loweralkylaralkyl; alkylthio loweralkyl; aralkyloxyalkyl; aralkylthioalkyl; or heteroaryloxyalkyl; wherein the latter 3 radicals may be substituted with halogen, loweralkyl, loweralkoxy, hydroxy, amino, aminomethyl, carboxyl, cyano and/or sulfamoyl; or alkenyl substituted by a heterocyclic group or alkyl substituted by a heterocyclic group, the latter two groups optionally being substituted by one or more groups chosen from loweralkyl, hydroxy, loweralkoxy, amino, loweralkylamino, di-loweralkylamino, acylamino, halogeno, halogenoloweralkyl, cyano and/or sulfonamide;

$R_2$ is —COOH, COO-(loweralkyl), —COO-(aryl lower alkyl), —COO aryl, or the group

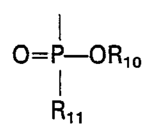

wherein

$R_{10}$ is hydrogen, lower alkyl or benzyl; and

$R_{11}$ is hydroxy, alkoxy, benzyloxy, lower alkyl, aryl or benzyl;

$R_3$ is hydrogen or lower alkyl;

X is 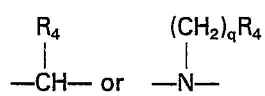

wherein q is 0 or 1;

$R_4$ and $R_5$ are chosen from the groups defined for $R_1$, indolyl (e.g. 3-indolyl), indolylalkyl (e.g.) 3-indolylalkyl), adamantyl (e.g. 1-adamantyl), adamantylmethyl (e.g. 1-adamantylmethyl) and amino methylphenylloweralkyl;

y is zero or an integer from 1 to 3;

$R_6$ is chosen from hydroxy, loweralkoxy and the group $—NR_8R_9$ in which $R_8$ and $R_9$ are independently hydrogen, aralkyl, or loweralkyl which together with the nitrogen atom to which they are attached may form a 4 to 6 membered heterocyclic group, one of whose members may be oxygen or sulphur;

and wherein, when X is

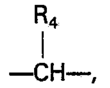

and y is zero, the following provisions apply:

(i) if $R_2$ is —COOH, —COO(loweralkyl), —COO(arylloweralkyl) or —COO aryl and $R_6$ is —OH, lower alkoxy, loweralkylamino, diloweralkylamino aralkylamino or diarylalkyl amino, then $R_4$ is chosen from G—A— in which G is 2- or 3-thienyl or 1- or 2-naphthyl and A is —$CH_2$— or —$CH_2$-lower alkyl-,

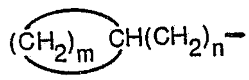

in which n is 0 or an integer from 1 to 8 and m is an integer from 2 to 8, adamantyl-methyl, unsubstituted or substituted phenyl, or substituted phenyl lower alkyl in which the substituent(s) for the latter two groups is or are chosen from halogen, trifluoromethyl, nitro, loweralkyl or loweralkoxy; further provided that

(ii) if, when $R_2$ and $R_6$ are as set forth in proviso (i), and $R_1$ is hydrogen or methyl, $R_3$ is hydrogen and $R_5$ is hydrogen, lower alkyl, unsubstituted phenyl, benzyl, benzyl substituted by halogen, hydroxyloweralkyl, loweralkoxyloweralkyl, phenoxy loweralkyl, mercapto loweralkyl, alkylthioloweralkyl, phenylthioloweralkyl or benzyl lower alkyl, then $R_4$ is chosen from G—A as defined above,

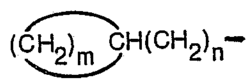

as defined above, (but excluding cyclohexyl and cyclohexylmethyl) adamantyl methyl, phenyl, or phenyl loweralkyl which latter two groups are substituted by nitro, loweralkyl or lower alkoxy;

(iii) if, when $R_1$, $R_2$, $R_3$ and $R_5$ are as set forth in proviso (ii) and $R_6$ is amino then $R_4$ cannot be the group —$(CH_2)_t$—$R_x$ in which t is 0 or 1 and $R_x$ is hydrogen, alkyl, cyclohexyl, phenyl or phenyl substituted by halogen, trifluoromethyl or hydroxy;

(iv) when $R_6$ is amino, $R_1$ is hydrogen, alkyl $C_1$—$C_{10}$, hydroxy-lower-alkyl, lower-alkoxy-lower-alkyl, aryloxy-lower-alkyl, heteroaryloxy-loweralkyl, amino-lower-alkyl, loweralkyl-aminoloweralkyl, diloweralkylamino-lower alkyl, acylaminoloweralkyl, arylaminoloweralkyl, guanidino-lower-alkyl, alkyl substituted by a heterocyclic group, alkylthioloweralkyl, arylthioloweralkyl, arylloweralkyl, aralkyloxyloweralkyl, aralkylthioloweralkyl, in which the aryl group of any foregoing aryl containing substituent may be substituted by halogen, loweralkyl, hydroxy, lower-alkoxy-amino, cyano or sulfonamido; $R_2$ is —COOH, —COO alkyl, COO aryl, —COO (arylloweralkyl)), $R_3$ is hydrogen, and $R_4$ is hydrogen, lower-alkyl, phenyl-lower-alkyl, amino-methylphenyllower-alkyl, hydroxy-phenylloweralkyl, hydroxyloweralkyl, acylaminoloweralkyl, aminoloweralkyl, dimethyl aminoloweralkyl, guanidinoloweralkyl, indolylalkyl, lower-alkylthio-lower-alkyl; then $R_5$ cannot be a group having the structure

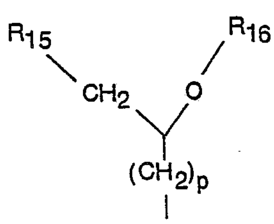 ; 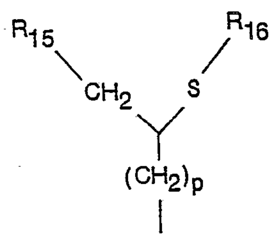 ; 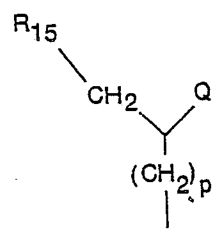 ;

wherein

p is 0, 1 or 2

$R_{15}$ is hydrogen or lower alkyl

$R_{16}$ is lower alkyl, aryl, aralkyl or heteroaryl and

Q is $C_3$—$C_8$ cycloalkyl; or the group

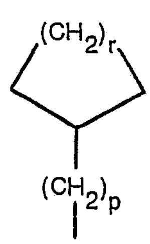

wherein p is as defined above and r is 2 or 3,

and wherein the terms alkyl, alkoxy and alkenyl and above refer to such groups having from 1 to 20 carbon atoms, said groups being straight chained, or branched chained and/or comprising one or more alicyclic rings; loweralkyl and loweralkoxy indicate such groups as aforesaid but having 1 to 8 carbon atoms.

2. A compound as defined in Claim 1, wherein:

X is 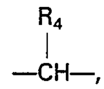

y is 1, 2 or 3, and $R_2$ is —COOH, —COO(loweralkyl), —COO(aryllowerakyl), or —COO(aryl), $R_3$ is hydrogen and $R_4$ and $R_5$ are chosen from hydrogen, the group G—A— as defined in Claim 1, 1-adamantylmethyl, cycloloweralkyl, hydroxyloweralkyl, alkyl, acylaminoloweralkyl, aminoloweralkyl, di-lower-alkylamino-loweralkyl, halogeno-loweralkyl, guanidino-loweralkyl, imidazolylloweralkyl, indolylloweralkyl, mercapto loweralkyl, loweralkylthioloweralkyl, unsubstituted phenyl, unsubstituted or substituted phenylloweralkyl wherein the substituent(s) for the latter two groups is or are chosen from halogeno, loweralkyl, lower-alkoxy, trifluoromethyl and nitro,

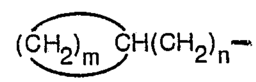

wherein n and m are as defined in Claim 1, and aminomethylphenylloweralkyl.

3. A compound as defined in Claim 1, in which

X is 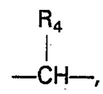

y is zero, and subject to provisions (i), (ii), (iii) and (iv) of Claim 1,

$R_1$ is H, alkyl, aralkyl, phenyl, aralkyloxyalkyl, or heteroaryloxyalkyl wherein the latter three groups are optionally substituted as defined in Claim 1,

$R_3$ is hydrogen;

$R_4$ is chosen from the group G—A— as defined in Claim 1,

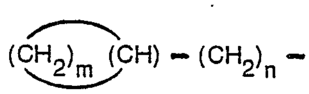

in which n and m are as defined in Claim 1, substituted or unsubstituted phenyl or substituted phenylloweralkyl in which the substituent(s) for the latter two groups is or are chosen from halogen, trifluoromethyl, nitro, loweralkyl, and loweralkoxy, with the proviso for $R_4$ only that when $R_6$ is the group

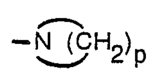 or 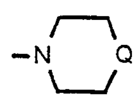

in which p is 3, 4 or 5, and Q is oxygen or sulfur, $R_4$ may also be hydrogen, loweralkyl, indolylloweralkyl, mercaptoloweralkyl, loweralkylthio-loweralkyl, guanidinoloweralkyl, imidazolyl-loweralkyl, hydroxy substituted phenyl,

$R_5$ is chosen from the groups defined for $R_4$ above, including those set forth in the proviso in this claim, and

$R_6$ is chosen from hydroxy, loweralkoxy or the group —NR'$_8$R'$_9$, wherein R'$_8$ and R'$_9$ independently are chosen from hydrogen, loweralkyl, or together with the nitrogen atom to which they are attached are

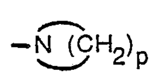 or 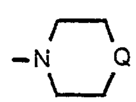

in which p and Q are as defined above.

4. A compound according to Claim 2 or 3, in which $R_1$ is chosen from loweralkyl and phenyl loweralkyl,

$R_2$ is chosen from —COOH and —COO-(loweralkyl),

$R_4$ is chosen from 2- or 3-thienylmethyl, 1- or 2-naphthylmethyl, benzyl or halogeno-substituted benzyl, and

$R_6$ is —OH or lower alkoxy.

5. A compound according to Claim 1, wherein y is 1, 2 or 3,

X is

$$-\overset{R_4}{\overset{|}{C}}H-$$

$R_1$ is hydrogen, phenylethyl, benzyl or loweralkylthioloweralkyl,
$R_2$ is carboxy or loweralkoxy carbonyl,
$R_3$ is hydrogen,
$R_4$ is benzyl or lower alkyl,
$R_5$ is hydrogen and
$R_6$ is hydroxy or loweralkoxy.

6. A compound according to Claim 1, the compound being

N-(L-1-ethoxycarbonylethyl)-L-2-thienyl-alanylglycine,
N-(L-1-ethoxycarbonylethyl)-L-2-thienyl-alanyl-L-alanine,
N-(L-1-ethoxycarbonylethyl)-L-3-thienyl-alanyl-L-alanine,
N-(L-1-ethoxycarbonylethyl)-L-3-thienyl-alanylglycine,
N-(L-1-carboxyethyl)-L-2-thienylalanyl-L-alanine,

or a pharmaceutically acceptable salt thereof.

7. A compound according to Claim 1, the compound being
N-(L-1-carboxy-3-phenylpropyl)-L-3-(3-thienyl)alanyl-L-alanine
N-(D,L-1-carboxy-3-phenylpropyl)-L-3-(2-naphthyl)alanyl-L-alanine
N-(L-1-ethoxycarbonyl-3-phenylpropyl)-L-3-(2′-thienyl)alanyl-L-alanine,
N-(D-1-carboxy-3-phenylpropyl)-L-(4-fluorophenyl)alanyl-L-alanine
N-(L-1-carboxy-3-phenylpropyl)-L-3-(1-naphthyl)alanyl-β-alanine,
N-(D-1-carboxy-3-phenylpropyl)-L-3-(3-thienyl)alanyl-L-alanine
N-(L-1-carboxy-2-(benzylthio)-ethyl)-L-phenylalanyl-β-alanine
N-(L-1-carboxy-2-phenethyl)-L-phenylalanyl-β-alanine
N-(L-1-carboxy-2-phenethyl)-L-phenylalanyl-δ-aminobutyric acid
N-(L-1-carboxy-3-phenylpropyl)-L-phenylalanyl-1-alanine hemihydrate or
N-(L-1-carboxy-3-phenylpropyl)-D,L-3-(1-naphthyl)alanyl-L-alanine
or a pharmaceutically acceptable salt thereof.

8. A compound according to Claim 1, in which y is zero,
$R_1$ is as defined in Claim 1,

$R_2$ is

$$O{=}\underset{R_{11}}{\underset{|}{\overset{|}{P}}}{-}OR_{10}$$

$R_3$, $R_4$, $R_5$, $R_{10}$ and $R_{11}$ and X are as defined in Claim 1, $R_6$ is —OH, provided that, when $R_4$ is hydrogen, aryl or heteroaryl, X is

$$-\overset{R_4}{\overset{|}{C}}H-.$$

9. A pharmaceutical composition comprising a compound of formula I as defined in Claim 1, or a salt thereof, mixed with a pharmaceutically acceptable carrier or excipient.

10. A pharmaceutical composition according to Claim 9, in which the compound is
N-(L-1-carboxy-3-phenylpropyl)-D,L-3-(1-naphthyl)alanyl-L-alanine, or
N-(L-1-carboxy-3-phenylpropyl)-L-phenyl-alanyl-β-alanine hemihydrate.

11. A compound of formula I as defined in Claim 1, including those compounds excluded from Claim 1 by proviso (i) but excluding those compound excluded from Claim 1 by provisos (ii), (iii) or (iv), for use in the inhibition of enkephalinase.

**Claims for the Contracting State: AT**

1. A method of preparing a compound having the formula:

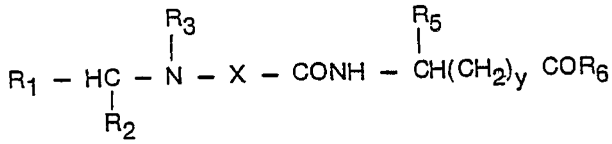

I

in which

$R_1$ is hydrogen; alkyl; halogen loweralkyl; hydroxy loweralkyl; loweralkoxy loweralkyl; aryloxy loweralkyl; amino loweralkyl; loweralkylamino loweralkyl; diloweralkyl aminoloweralkyl; acylamino loweralkyl; diarylaminoloweralkyl; arylamino loweralkyl; guanidino loweralkyl; heteroaryl; aryl, aralkyl; mercapto loweralkyl; arylthio loweralkyl; loweralkylaralkyl; alkylthio loweralkyl; aralkyloxyalkyl; aralkylthioalkyl; or heteroaryloxyalkyl; wherein the latter 3 radicals may be substituted with halogen, loweralkyl, loweralkoxy, hydroxy, amino, aminomethyl, carboxyl, cyano and/or sulfamoyl; or alkenyl substituted by a heterocyclic group or alkyl substituted by a heterocyclic group, the latter two groups optionally being substituted by one or more groups chosen from loweralkyl, hydroxy, loweralkoxy, amino, loweralkylamino, di-loweralkylamino, acylamino, halogeno, halogenoloweralkyl, cyano and/or sulfonamide;

$R_2$ is —COOH, COO-(loweralkyl), —COO-(aryl lower alkyl), —COO aryl, or the group

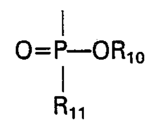

wherein

$R_{10}$ is hydrogen, lower alkyl or benzyl; and

$R_{11}$ is hydroxy, alkoxy, benzyloxy, lower alkyl, aryl or benzyl;

$R_3$ is hydrogen or lower alkyl;

X is 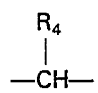 or 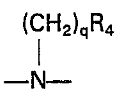

wherein q is 0 or 1;

$R_4$ and $R_5$ are chosen from the groups defined for $R_1$, indolyl (e.g. 3-indolyl), indolylalkyl (e.g.) 3-indolylalkyl), adamantyl (e.g. 1-adamantyl), adamantylmethyl (e.g. 1-adamantylmethyl) and amino methylphenylloweralkyl;

y is zero or an integer from 1 to 3; and

$R_6$ is chosen from hydroxy, loweralkoxy and the group $—NR_8R_9$ in which $R_8$ and $R_9$ are independently hydrogen, aralkyl, or loweralkyl which together with the nitrogen atom to which they are attached may form a 4 to 6 membered heterocyclic group, one of whose members may be oxygen or sulphur;

and wherein, when X is

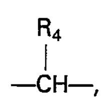

and y is zero, the following provisions apply:

(i) if $R_2$ is —COOH, —COO(loweralkyl), —COO(aryllloweralkyl) or COO aryl and $R_6$ is —OH, lower alkoxy, loweralkylamino, dilòweralkylamino aralkylamino or diarylalkyl amino, then $R_4$ is chosen from G—A— in which G is 2- or 3-thienyl or 1- or 2-naphthyl and A is $—CH_2—$ or $—CH_2$-lower alkyl-,

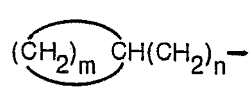

in which n is 0 or an integer from 1 to 8 and m is an integer from 2 to 8, adamantylmethyl, unsubstituted or substituted phenyl, or substituted phenyl lower alkyl in which the substituent(s) for the latter two groups is or are chosen from halogen, trifluoromethyl, nitro, loweralkyl or loweralkoxy; further provided that

(ii) if, when $R_2$ and $R_6$ are as set forth in proviso (i), and $R_1$ is hydrogen or methyl, $R_3$ is hydrogen and $R_5$ is hydrogen, lower alkyl, unsubstituted phenyl, benzyl, benzyl substituted by halogen, hydroxyloweralkyl,

loweralkoxyloweralkyl, phenoxy loweralkyl, mercapto loweralkyl, alkylthioloweralkyl, phenylthioloweralkyl or benzyl lower alkyl, then $R_4$ is chosen from G—A as defined above,

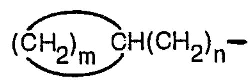

as defined above, (but excluding cyclohexyl and cyclohexylmethyl) adamantyl methyl, phenyl, or phenyl loweralkyl which latter two groups are substituted by nitro, loweralkyl or lower alkoxy;

(iii) if, when $R_1$, $R_2$, $R_3$ and $R_5$ are as set forth in proviso (ii) and $R_6$ is amino then $R_4$ cannot be the group $-(CH_2)_t-R_x$ in which t is 0 or 1 and $R_x$ is hydrogen, alkyl, cyclohexyl, phenyl or phenyl substituted by halogen, trifluoromethyl or hydroxy;

(iv) when $R_6$ is amino, $R_1$ is hydrogen, alkyl $C_1$—$C_{10}$, hydroxy-lower-alkyl, lower-alkoxy-lower-alkyl, aryloxy-lower-alkyl, heteroaryloxy-loweralkyl, amino-lower-alkyl, loweralkyl-aminoloweralkyl, diloweralkylamino-lower alkyl, acylaminoloweralkyl, arylaminoloweralkyl, guanidino-lower-alkyl, alkyl substituted by a heterocyclic group, alkylthioloweralkyl, arylthioloweralkyl, arylloweralkyl, aralkyloxyloweralkyl, aralkylthioloweralkyl, in which the aryl group of any foregoing aryl containing substituent may be substituted by halogen, loweralkyl, hydroxy, lower-alkoxy-amino, cyano or sulfonamido; $R_2$ is —COOH, —COO alkyl, COO aryl, —COO (arylloweralkyl)), $R_3$ is hydrogen, and $R_4$ is hydrogen, lower-alkyl, phenyl-lower-alkyl, amino-methylphenyllower-alkyl, hydroxy-phenylloweralkyl, hydroxyloweralkyl, acylaminoloweralkyl, aminoloweralkyl, dimethyl aminoloweralkyl, guanidinoloweralkyl, indolylalkyl, lower-alkylthio-lower-alkyl; then $R_5$ cannot be a group having the structure

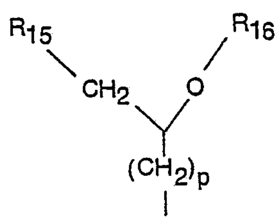 ; 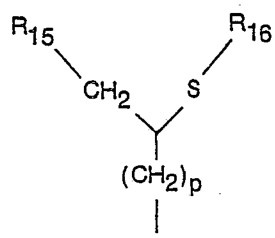 ; 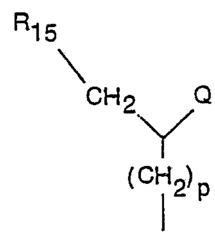 ;

wherein

p is 0, 1 or 2

$R_{15}$ is hydrogen or lower alkyl

$R_{16}$ is lower alkyl, aryl, aralkyl or heteroaryl and

Q is $C_3$—$C_8$ cycloalkyl; or the group

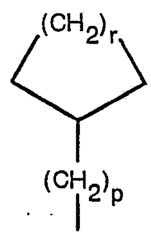

wherein p is as defined above and r is 2 or 3,

or a pharmaceutically acceptable salt thereof, characterised in that the compound is prepared by an appropriate method selected from the following methods (wherein in the following formulae $R_1$, $R_2$, $R_3$, $R_4$, $R_5$, $R_6$, $R_{10}$, $R_{11}$ and y are as defined for formula I, including suitable protection of any reactive groups):

a) (for the preparation of compounds of formula I in which $R_3$ is hydrogen and $R_2$ is o?her than

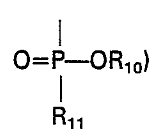

reducing a compound of the formula A

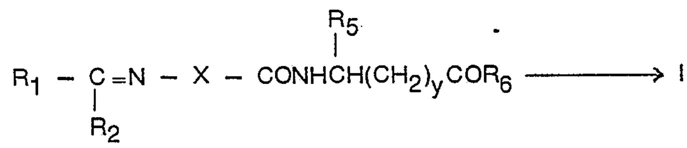

*A*

b) coupling an amino acid A′ with an amino acid B′

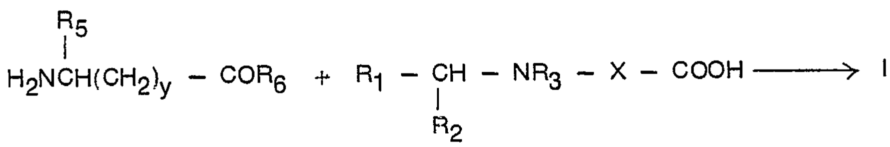

*A′* *B′*

c) (for the preparation of compounds of formula I in which $R_3$ is hydrogen) reducing a compound of the formula X, at the C=N double bond,

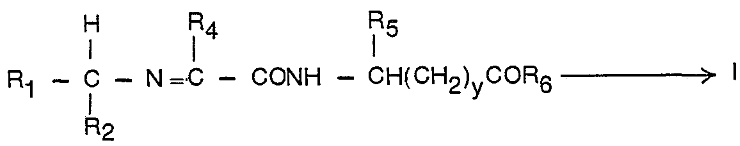

d) alkylating an amine of formula S′ with a compound of the formula T′

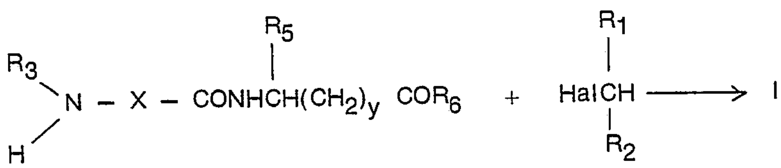

*S′* *T′*

in which Hal represents halogen.

e) (for the preparation of compounds in which $R_3$ is hydrogen and $R_2$ is

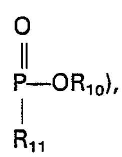

reacting a compound of formula A″ with a phosphite ester or phosphonate ester of the formula Z

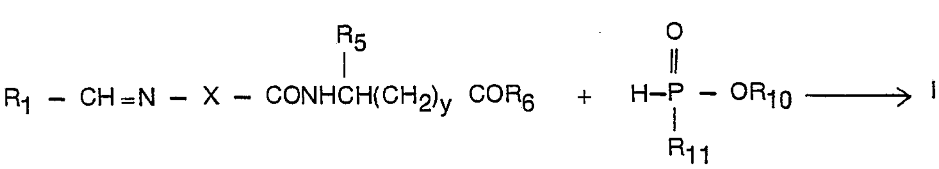

*A″* *Z*

followed by removal of any protecting groups if necessary to obtain the desired compound of formula I, and thereafter, if desired, converting the compound of formula I into another compound of formula I and/or

forming a salt thereof, and if desired isolating a preferred isomer;
and wherein the terms alkyl, alkoxy and alkenyl used above refer to such groups having from 1 to 20 carbon atoms, said groups being straight chained, or branched chained and/or comprising one or more alicyclic rings; loweralkyl and loweralkoxy indicate such groups as aforesaid but having 1 to 8 carbon atoms.

2. A method as defined in Claim 1 a) to d), characterized in that a compound is produced in which

X is 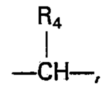

y is 1, 2 or 3, and $R_2$ is —COOH, —COO(loweralkyl), —COO(arylloweralkyl), or —COO(aryl), $R_3$ is hydrogen and $R_4$ and $R_5$ are chosen from hydrogen, the group G—A— as defined in Claim 1, 1-adamantylmethyl, cycloloweralkyl, hydroxyloweralkyl, alkyl, acylaminoloweralkyl, aminoloweralkyl, di-lower-alkylamino-loweralkyl, halogeno-loweralkyl, guanidino-loweralkyl, imidazolylloweralkyl, indolylloweralkyl, mercapto loweralkyl, loweralkylthioloweralkyl, unsubstituted phenyl, unsubstituted or substituted phenylloweralkyl wherein the substituent(s) for the latter two groups is or are chosen from halogeno, loweralkyl, lower-alkoxy, trifluoromethyl and nitro,

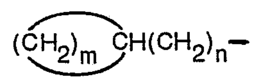

wherein n and m are as defined in Claim 1, and aminomethyl-phenylloweralkyl.

3. A method as defined in Claim 1, characterized in that a compound is produced in which

X is 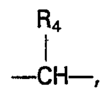

y is zero, and subject to provisions (i), (ii), (iii) and (iv) of Claim 1,

$R_1$ is H, alkyl, aralkyl, phenyl, aralkyloxyalkyl, or heteroaryloxyalkyl wherein the latter three groups are optionally substituted as defined in Claim 1,

$R_3$ is hydrogen;

$R_4$ is chosen from G—A— as defined in Claim 1,

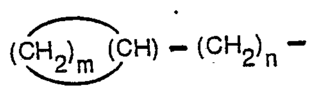

in which n and m are as defined in Claim 1, substituted or unsubstituted phenyl or substituted phenylloweralkyl in which the substituent(s) for the latter two groups is or are chosen from halogen, trifluoromethyl, nitro, loweralkyl, and loweralkoxy, with the proviso for $R_4$ only that when $R_6$ is the group

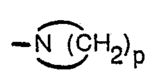 or 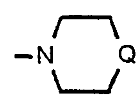

in which p is 3, 4 or 5, and Q is oxygen or sulfur, $R_4$ may also be hydrogen, loweralkyl, indolylloweralkyl, mercaptoloweralkyl, loweralkylthio-loweralkyl, guanidinoloweralkyl, imidazolyl-loweralkyl, hydroxy substituted phenyl,

$R_5$ is chosen from the groups defined for $R_4$ above, including those set forth in the proviso in this claim, and

$R_6$ is chosen from hydroxy, loweralkoxy or the group $—NR'_8R'_9$, wherein $R'_8$ and $R'_9$ independently are chosen from hydrogen, loweralkyl, or together with the nitrogen atom to which they are attached are

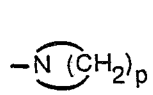 or 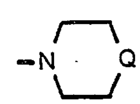

in which p and Q are as defined above.

4. A method as defined in Claim 2 or 3, characterized in that a compound is produced in which $R_1$ is chosen from loweralkyl and phenyl loweralkyl,

$R_2$ is chosen from —COOH and —COO-(loweralkyl),

$R_4$ is chosen from 2- or 3-thienylmethyl, 1- or 2-naphthylmethyl, benzyl or halogeno-substituted benzyl, and

$R_6$ is —OH or lower alkoxy.

5. A method as defined in Claim 1 a) to d), characterized in that a compound is produced in which y is 1, 2 or 3,

X is 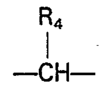

$R_1$ is hydrogen, phenylethyl, benzyl or loweralkylthioloweralkyl,
$R_2$ is carboxy or loweralkoxy carbonyl,
$R_3$ is hydrogen,
$R_4$ is benzyl or lower alkyl,
$R_5$ is hydrogen and
$R_6$ is hydroxy or loweralkoxy.

6. A method as defined in Claim 1 a) to d) characterized in that the compound

N-(L-1-ethoxycarbonylethyl)-L-2-thienyl-alanylglycine,
N-(L-1-ethoxycarbonylethyl)-L-2-thienyl-alanyl-L-alanine,
N-(L-1-ethoxycarbonylethyl)-L-3-thienyl-alanyl-L-alanine,
N-(L-1-ethoxycarbonylethyl)-L-3-thienyl-alanylglycine,
N-(L-1-carboxyethyl)-L-2-thienylalanyl-L-alanine,

or a pharmaceutically acceptable salt thereof is produced.

7. A method as defined in Claim 1 a) to d) characterized in that the compound

N-(L-1-carboxy-3-phenylpropyl)-L-3-(3-thienyl)alanyl-L-alanine
N-(D,L-1-carboxy-3-phenylpropyl)-L-3-(2-naphthyl)alanyl-L-alanine
N-(L-1-ethoxycarbonyl-3-phenylpropyl)-L-3-(2′-thienyl)alanyl-L-alanine,
N-(D-1-carboxy-3-phenylpropyl)-L-(4-fluorophenyl)alanyl-L-alanine
N-(L-1-carboxy-3-phenylpropyl)-L-3-(1-naphthyl)alanyl-β-alanine,
N-(D-1-carboxy-3-phenylpropyl)-L-3-(3-thienyl)alanyl-L-alanine
N-(L-1-carboxy-2-(benzylthio)-ethyl)-L-phenylalanyl-β-alanine
N-(L-1-carboxy-2-phenethyl)-L-phenylalanyl-β-alanine
N-(L-1-carboxy-2-phenethyl)-L-phenylalanyl-δ-aminobutyric acid
N-(L-1-carboxy-3-phenylpropyl)-L-phenylalanyl-β-alanine hemihydrate or
N-(L-1-carboxy-3-phenylpropyl)-D,L-3-(1-naphthyl)alanyl-L-alanine

or a pharmaceutically acceptable salt thereof is produced.

8. A method as defined in Claim 1 b) to e) characterized in that a compound is produced in which y is zero,

$R_1$ is as defined in Claim 1,

$R_2$ is 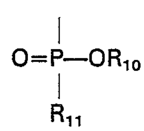

$R_3$, $R_4$, $R_5$, $R_{10}$ and $R_{11}$ and X are as defined in Claim 1, $R_6$ is —OH, provided that, when $R_4$ is hydrogen, aryl or heteroaryl, X is

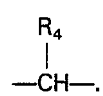

9. A method according to Claim 1, wherein method a) comprises condensing a keto acid or ester of formula *D*

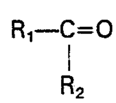 *D*

with an amino acid or ester of formula *C*

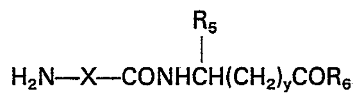

*C*

to form the compound of formula A, in a reactive medium containing a reducing agent so as to effect the reduction of the compound of formula A to the compound of formula I in said reactive medium as it is formed therein by the aforesaid condensation.

10. A method according to Claim 1, wherein method c) comprises condensing a keto acid or ester of formula E'

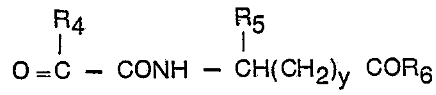

*E'*

with an amino acid or ester of formula F'

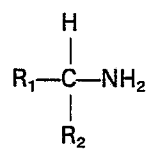

*F'*

to form the compound of formula X, in a reactive medium containing a reducing agent so as to effect the reduction of the compound of formula X to the compound of formula I in said reactive medium as it is formed by the aforesaid condensation.

11. A method of producing a pharmaceutical composition which comprises mixing a compound of formula I as defined in Claim 1, or a salt thereof with a pharmaceutically acceptable carrier or excipient.

12. A method of producing a pharmaceutical composition which comprises mixing a compound of formula I or salt thereof, prepared by a process of any one of Claims 1 to 10, with a pharmaceutically acceptable carrier or excipient.

**Patentansprüche für die Vertragsstaaten: BE CH DE FR GB IT LI NL SE**

1. Verbindung der Formel

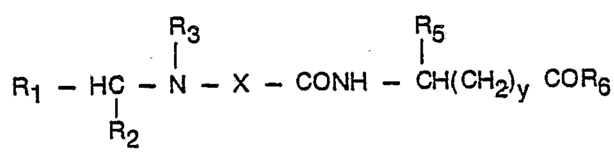

I

un deren pharmazeutisch annehmbaren Salze, worin

$R_1$ Wasserstoff; Alkyl; Halogen-niedrigalkyl; Hydroxy-niedrigalkyl; Niedrigalkoxy-niedrigalkyl; Aryloxy-niedrigalkyl; Amino-niedrigalkyl; Niedrigalkylamino-niedrigalkyl; Di-niedrigalkylamino-niedrigalkyl; Acylamino-niedrigalkyl; Diarylamino-niedrigalkyl; Arylaminoniedrigalkyl; Guanidino-niedrigalkyl; Heteroaryl; Aryl; Aralkyl; Mercapto-niedrigalkyl; Arylthio-niedrigalkyl; Niedrigalkyl-aralkyl; Alkylthio-niedrigalkyl; Aralkyloxyalkyl; Aralkylthioalkyl; oder Heteroaryloxyalkyl; wobei die drei letztgenannten Radikale durch Halogen, Niedrigalkyl, Niedrigalkoxy, Hydroxy, Amino, Aminomethyl, Carboxyl, Cyano und/oder Sulfamoyl substituiert sein können; oder durch einen heterocyclische Gruppe substituiertes Alkenyl oder durch eine heterocyclische Gruppe substituiertes Alkyl ist, wobei die beiden letztgenannten Gruppen durch eine oder mehrere der folgenden Gruppen: Niedrigalkyl, Hydroxy, Niedrigalkoxy, Amino, Niedrigalkylamino, Diniedrigalkylamino, Acylamino, Halogen, Halogen-niedrigalkyl, Cyano und/oder Sulfonamid substituiert sein können;

$R_2$ —COOH, COO-(Niedrigalkyl), —COO—(Aryl-niedrigalkyl), —COO-Aryl oder die Gruppe

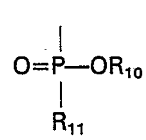

bedeutet worin

$R_{10}$ Wasserstoff, Niedrigalkyl oder Benzyl und

$R_{11}$ Hydroxy, Alkoxy, Benzyloxy, Niedrigalkyl, Aryl oder Benzyl darstellen;

$R_2$ Wasserstoff oder Niedrigalkyl ist;

X 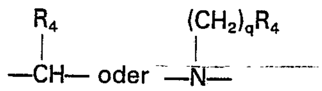

bedeutet, worin q 0 oder 1 ist;

$R_4$ und $R_5$ aus den in der Definition von $R_1$ erwähnten Gruppen sowie Indolyl (z.B. 3-Indolyl), Indolylalkyl (z.B. 3-Indolylalkyl), Adamantyl (z.B. 1-Adamantyl), Adamantylmethyl (z.B. 1-Adamantylmethyl) und Aminomethylphenyl-niedrigalkyl ausgewählt sind;

y 0 oder eine ganze Zahl von 1 bis 3 ist;

$R_6$ Hydroxy, Niedrigalkoxy oder die Gruppe $-NR_8R_9$ bedeutet, wobei $R_8$ und $R_9$ unabhängig Wasserstoff, Aralkyl oder Niedrigalkyl darstellen oder zusammen mit dem Stickstoffatom, an das sie gebunden sind, eine 4- bis 6-gleidrige heterocyclische Gruppe bilden, von der ein Glied Sauerstoff oder Schwefel sein kann;

wobei, wenn X für

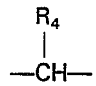

steht und y 0 ist, die folgenden Vorbehalte gelten:

(i) Wenn $R_2$ —COOH, —COO(Niedrigalkyl), —COO(Arylniedrigalkyl) oder —COO(Aryl) und $R_6$ —OH, Niedrigalkoxy, Niedrigalkylamino, Di-niedrigalkylamino, Aralkylamino oder Diaralkylamino sind, dann ist $R_4$ ausgewählt aus G—A—, worin G 2- oder 3-Thienyl oder 1- oder 2-Naphthyl und A —$CH_2$— oder —$CH_2$-Niedrigalkyl-,

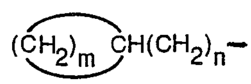

(worin n 0 oder eine ganze Zahl von 1 bis 8 und m eine ganze Zahl von 2 bis 8 sind), Adamantyl-methyl, unsubstituiertes oder substituiertes Phenyl oder substituiertes Phenyl-niedrigalkyl sind, wobei die Substituenten der beiden letzten Gruppen Halogen, Trifluoromethyl, Nitro, Niedrigalkyl oder Niedrigalkoxy sein können;

(ii) wenn $R_2$ und $R_6$ die unter (i) genannten Bedeutungen haben und $R_1$ Wasserstoff oder Methyl, $R_3$ Wasserstoff und $R_5$ WAasserstoff, Niedrigalkyl, unsubstituiertes Phenyl, Benzyl, halogen-substituiertes Benzyl, Hydroxy-niedrigalkyl, Niedrigalkoxy-niedrigalkyl, Phenoxy-niedrigalkyl, Mercapto-niedrigalkyl, Alkylthio-niedrigalkyl, Phenylthioniedrigalkyl oder Benzyl-niedrigalkyl sind, dann ist $R_4$ ausgewählt aus G—A (wie oben definiert),

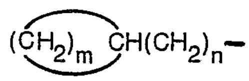

(wie oben definiert, ausgenommen Cyclohexyl und Cyclohexylmethyl), Adamantylmethyl, Phenyl oder Phenyl-niedrigalkyl, wobei die beiden letztgenannten Gruppen durch Nitro, Niedrigalkyl oder Niedrigalkoxy Substituiert sind;

(iii) wenn $R_1$, $R_2$, $R_3$ und $R_5$ die unter (ii) genannten Bedeutungen haben und $R_6$ Amino ist, dann kann $R_4$ nicht die Gruppe $-(CH_2)_t-R_x$ sein, worin t 0 oder 1 und $R_x$ Wasserstoff, Alkyl, Cyclohexyl, Phenyl oder durch Halogen, Trifluoromethyl oder Hydroxy substituiertes Phenyl sind;

(iv) wenn $R_6$ Amino, $R_1$ Wasserstoff, $C_1$—$C_{10}$-Alkyl, Hydroxy-niedrigalkyl, Niedrigalkoxy-niedrigalkyl, Aryloxy-niedrigalkyl, Heteroaryloxy-niedrigalkyl, Amino-niedrigalkyl, Niedrigalkylamino-niedrigalkyl, Di-niedrigalkylamino-niedrigalkyl, Acylamino-niedrigalkyl, Arylamino-niedrigalkyl, Guanidino-niedrigalkyl, durch eine heterocyclische Gruppe substituiertes Alkyl, Alkylthio-niedrigalkyl, Arylthio-niedrigalkyl, Aryl-niedrigalkyl, Aralkyloxy-niedrigalkyl, Aralkylthio-niedrigalkyl (wobei die Arylgruppe jedes vorstehend genannten arylhaltigen Substituenten durch Halogen, Niedrigalkyl, Hydroxy, Niedrigalkoxy-amino, Cyano oder Sulfonamido substituiert sein kann), $R_2$ —COOH, —COO-Alkyl, —COO-Aryl, —COO-(Aryl-niedrigalkyl), $R_3$ Wasserstoff und $R_4$ Wasserstoff, Niedrigalkyl, Phenyl-niedrigalkyl, Aminomethylphenyl-niedrigalkyl, Hydroxyphenyl-niedrigalkyl, Hydroxy-niedrigalkyl, Acylamino-niedrigalkyl, Amino-niedrigalkyl, Dimethylamino-niedrigalkyl, Guanidino-niedrigalkyl, Indolylalkyl oder Niedrigalkylthio-niedrigalkyl sind, dann kann $R_5$ keine der Gruppen mit der Struktur

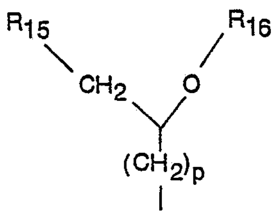 ; 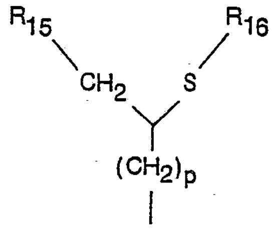 ; 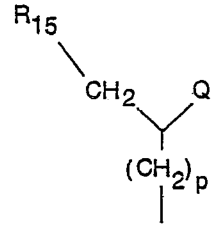 ;

worin
p 0, 1 oder 2,
$R_{15}$ Wasserstoff oder Niedrigalkyl,
$R_{16}$ Niedrigalkyl, Aryl, Aralkyl oder Heteroaryl und
Q $C_3$—$C_8$ Cycloalkyl sind, oder

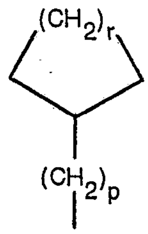

sein, worin p wie oben definiert und r 2 oder 3 sind und worin die Ausdrücke Alkyl, Alkoxy und Alkenyl sich auf Gruppen mit 1—20 Kohlenstoffatomen beziehen, die geradkettig, verzweigtkettig sein oder eine oder mehrere alicyclische Ringe enthalten können und worin Niedrigalkyl und Niedrigalkoxy solche Gruppen mit 1 bis 8 Kohlenstoffatomen bedeuten.

2. Verbindung nach Anspruch 1, worin

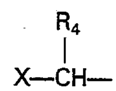

ist, y 1, 2 oder 3 ist, $R_2$ —COOH, —COO-(Niedrigalkyl), —COO(Arylniedrigalkyl) oder —COO-(Aryl) bedeutet, $R_3$ Wasserstoff ist und $R_4$ und $R_5$ aus Wasserstoff, der Gruppe G—A— (wie in Anspruch 1 definiert), 1-Adamantylmethyl, Cycloniedrigalkyl, Hydroxyniedrigalkyl, Alkyl, Acylaminoniedrigalkyl, Amino-niedrigalkyl, Di-Niedrigalkylamino-niedrigalkyl, Halogeno-niedrigalkyl, Guanidino-niedrigalkyl, Imidazolylniedrigalkyl, Indolyl-niedrigalkyl, Mercapto-niedrigalkyl, Niedrigalkylthio-niedrigalkyl, unsubstituiertem Phenyl, unsubstituiertem oder substituiertem Phenyl-niedrigalkyl (wobei die Substituenten der beiden letztgenannten Gruppen Halogeno, Niedrigalkyl, Niedrigalkoxy, Trifluoromethyl oder Nitro sein können),

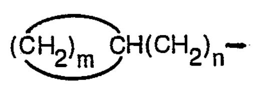

(worin n und m wie in Anspruch 1 definiert sind) und Aminomethyl-phenylniedrigalkyl ausgewählt sind.

3. Verbindung nach Anspruch 1, worin

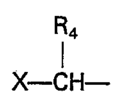

und y null und den Vorbehalten (i), (ii), (iii) und (iv) des Anspruchs 1 unterworfen sind,

$R_1$ H, Alkyl, Aralkyl, Phenyl, Aralkyloxyalkyl oder Heteroaryloxyalkyl ist, wobei die drei letztgenannten Gruppen wie in Anspruch 1 definiert substituiert sein können,

$R_3$ Wasserstoff;

$R_4$ aus der Gruppe G—A— (wie in Anspruch 1 definiert), der Gruppe

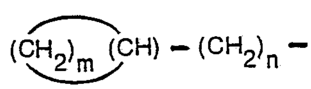

(worin n und m wie in Anspruch 1 definiert sind), substituiertem oder unsubstituiertem Phenyl sowie substituiertem Phenyl-niedrigalkyl ausgewählt ist, wobei die Substituenten der beiden letztgenannten

Gruppen Halogen, Trifluoromethyl, Nitro, Niedrigalkyl oder Niedrigalkoxy sein können, mit dem nur für $R_4$ geltenden Vorbehalt, dass dieses, wenn $R_6$

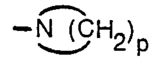 oder 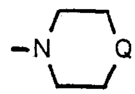

ist, worin p 3, 4 oder 5 und Q Sauerstoff oder Schwefel sind, auch Wasserstoff, Niedrigalkyl, Indolyl-niedrigalkyl, Mercapto-niedrigalkyl, Niedrigalkylthio-niedrigalkyl, Guanidino-niedrigalkyl, Imidazolylniedrigalkyl, hydroxy-substituiertes Phenyl sein kann;

$R_5$ aus den für $R_4$ definierten Gruppen einschliesslich der in dem Vorbehalt genannten; und

$R_6$ aus Hydroxy, Niedrigalkoxy oder der Gruppe $-NR'_8R'_9$ ausgewählt sind, worin $R'_8$ und $R'_9$ unabhängig Wasserstoff oder Niedrigalkyl sind oder zusammen mit dem Stockstoffatom, an das sie gebunden sind, die Gruppe

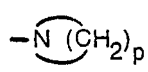 oder 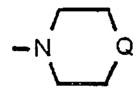

bilden, worin p und Q wie oben definiert sind.

4. Verbindung nach Anspruch 2 oder 3, worin $R_1$ Niedrigalkyl oder Phenylniedrigalkyl,
$R_2$ $-COOH$ oder $-COO$-(Niedrigalkyl),
$R_5$ 2- oder 3-Thienylmethyl, 1- oder 2-Naphthylmethyl, Benzyl oder halogensubstituiertes Benzyl und
$R_6$ $-OH$ oder Niedrigalkoxy sind.

5. Verbindung nach Anspruch 1, worin y 1, 2 oder 3,

X $$-\overset{\overset{R_4}{|}}{C}H-,$$

$R_1$ Wasserstoff, Phenyläthyl, Benzyl oder Niedrigalkylthio-niedrigalkyl,
$R_2$ Carboxy oder Niedrigalkoxy-carbonyl,
$R_3$ Wasserstoff,
$R_4$ Benzyl oder Niedrigalkyl,
$R_5$ Wasserstoff und
$R_6$ Hydroxy oder Niedrigalkoxy sind.

6. Verbindung nach Anspruch 1, und zwar
N-(L-1-Aethoxycarbonyläthyl)-L-2-thienyl-alanyl-glycin,
N-(L-1-Aethoxycarbonyläthyl)-L-2-thienyl-alanyl-L-alanin,
N-(L-1-Aethoxycarbonyläthyl)-L-3-thienyl-alanyl-L-alanin,
N-(L-1-Aethoxycarbonyläthyl)-L-3-thienyl-alanyl-glycin,
N-(L-1-Carboxyäthyl)-L-2-thienylalanyl-L-alanin
oder ein pharmazeutisch annehmbares Salz davon.

7. Verbindung nach Anspruch 1, und zwar
N-(L-1-Carboxy-3-phenylpropyl)-L-3-(3-thienyl)alanyl-L-alanin,
N-(D,L-1-Carboxy-3-phenylpropyl)-L-3-(2'-naphthyl)alanyl-L-alanin,
N-(L-1-Aethoxycarbonyl-3-phenylpropyl)-L-3-(2'-thienyl)-alanyl-L-alanin,
N-(D-1-Carboxy-3-phenylpropyl)-L-(4-fluorophenyl)alanyl-L-alanin,
N-(L-1-Carboxy-3-phenylpropyl)-L-3-(1-naphthyl)alanyl-β-alanin,
N-(D-1-Carboxy-3-phenylpropyl)-L-3-(3-thienyl)alanyl-L-alanin,
N-(L-1-Carboxy-2-(benzylthio)-äthyl)-L-phenylalanyl-β-alanin,
N-(L-1-Carboxy-2-phenäthyl)-L-phenylalanyl-β-alanin,
N-(L-1-Carboxy-2-phenäthyl)-L-phenylalanyl-γ-aminobuttersäure,
N-(L-1-Carboxy-3-phenylpropyl)-L-phenylalanyl-β-alanin-Hemihydrat oder
N-(L-1-Carboxy-3-phenylpropyl)-D,L-3-(1-naphthyl)alanyl-L-alanin
oder ein pharmazeutisch annehmbares Salz davon.

8. Verbindung nach Anspruch 1, worin y null, $R_1$ wie in Anspruch 1 definiert,,,

$R_2$ $$O{=}\underset{\underset{R_{11}}{|}}{\overset{|}{P}}{-}OR_{10},$$

$R_3$, $R_4$, $R_5$, $R_{10}$, $R_{11}$ und X wie in Anspruch 1 definiert und $R_6$ —OH sind, mit dem Vorbehalt, dass

X 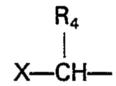

ist, wenn $R_4$ Wasserstoff, Aryl oder Heteroaryl ist.

9. Pharmazeutische Zubereitung, enthaltend eine Verbindung der Formel I, wie in Anspruch 1 definiert, oder ein Salz davon, gemischt mit einem pharmazeutisch annehmbaren Trägerstoff oder Exzipienten.

10. Pharmazeutische Zubereitung nach Anspruch 9, worin die Verbindung N-(L-1-Carboxy-3-phenylpropyl)-D,L-3-(1-naphthyl)alanyl-L-alanin oder N-(L-1-Carboxy-3-phenylpropyl)-L-phenyl-alanyl-β-alanin-Hemihydrat ist,

11. Verbindung der Formel I, wie in Anspruch 1 definiert, einschliesslich der aus Anspruch 1 durch Vorbehalt (i), aber ausschliesslich der aus Anspruch 1 durch Vorbehalte (ii), (iii) oder (iv) ausgeschlossenen Verbindung, zur Verwendung für die Inhibierung von Enkephalinase.

**Patentansprüche für den Vertragsstaat: AT**

1. Verfahren zur Herstellung von Verbindungen der Formel

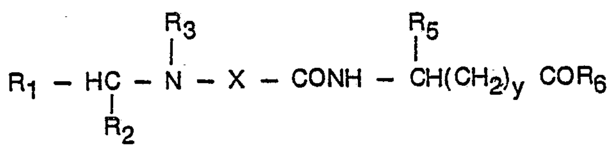 I

und deren pharmazeutisch annehmbaren Salzen, worin

$R_1$ Wasserstoff; Alkyl; Halogen-niedrigalkyl; Hydroxy-niedrigalkyl; Niedrigalkoxy-niedrigalkyl; Aryloxy-niedrigalkyl; Amino-niedrigalkyl; Niedrigalkylamino-niedrigalkyl; Di-niedrigalkylamino-niedrigalkyl; Acylamino-niedrigalkyl; Diarylamino-niedrigalkyl; Arylaminoniedrigalkyl; Guanidino-niedrigalkyl; Heteroaryl; Aryl; Aralkyl; Mercapto-niedrigalkyl; Arylthio-niedrigalkyl; Niedrigalkyl-aralkyl; Alkylthio-niedrigalkyl; Aralkyloxyalkyl; Aralkylthioalkyl; oder Heteroaryloxyalkyl; wobei die drei letztgenannten Radikale durch Halogen, Niedrigalkyl, Niedrigalkoxy, Hydroxy, Amino, Aminomethyl, Carboxyl, Cyano und/oder Sulfamoyl substituiert sein können; oder durch eine heterocyclische Gruppe substituiertes Alkenyl oder durch eine heterocyclische Gruppe substituiertes Alkyl ist, wobei die beiden letztgenannten Gruppen durch eine oder mehrere der folgenden Gruppen: Niedrigalkyl, Hydroxy, Niedrigalkoxy, Amino, Niedrigalkylamino, Diniedrigalkylamino, Acylamino, Halogen, Halogen-niedrigalkyl, Cyano und/oder Sulfonamid substituiert sein können;

$R_2$ —COOH, COO-(Niedrigalkyl), —COO—(Aryl-niedrigalkyl), —COO-Aryl oder die Gruppe

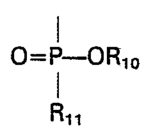

bedeutet worin

$R_{10}$ Wasserstoff, Niedrigalkyl oder Benzyl und

$R_{11}$ Hydroxy, Alkoxy, Benzyloxy, Niedrigalkyl, Aryl oder Benzyl darstellen;

$R_3$ Wasserstoff oder Niedrigalkyl ist;

X 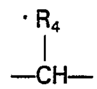 oder 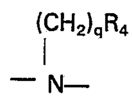

bedeutet, worin q 0 oder 1 ist;

$R_4$ und $R_5$ aus den in der Definition von $R_1$ erwähnten Gruppen sowie Indolyl (z.B. 3-Indolyl), Indolylalkyl (z.B. 3-Indolylalkyl), Adamantyl (z.B. 1-Adamantyl), Adamantylmethyl (z.B. 1-Adamantylmethyl) und Aminomethylphenyl-niedrigalkyl ausgewählt sind;

y 0 oder eine ganze Zahl von 1 bis 3 ist;

$R_6$ Hydroxy, Niedrigalkoxy oder die Gruppe —$NR_8R_9$ bedeutet, wobei $R_8$ und $R_9$ unabhängig Wasserstoff, Aralkyl oder Niedrigalkyl darstellen oder zusammen mit dem Stickstoffatom, an das sie gebunden sind, eine 4- bis 6-gliedrige heterocyclische Gruppe bilden, von der ein Glied Sauerstoff oder Schwefel sein kann;

wobei, wenn X für 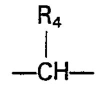 steht und y 0 ist, die folgenden Vorbehalte gelten:

(i) Wenn $R_2$ —COOH, —COO(Niedrigalkyl), —COO(Arylniedrigalkyl) oder —COO(Aryl) und $R_6$ —OH, Niedrigalkoxy, Niedrigalkylamino, Di-niedrigalkylamino, Aralkylamino oder Diaralkylamino sind, dann ist $R_4$ ausgewählt aus G—A—, worin G 2- oder 3-Thienyl oder 1- oder 2-Naphthyl und A —$CH_2$— oder —$CH_2$-Niedrigalkyl-,

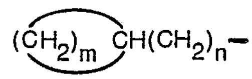

(worin n 0 oder eine ganze Zahl von 1 bis 8 und m eine ganze Zahl von 2 bis 8 sind), Adamantyl-methyl, unsubstituiertes oder substituiertes Phenyl oder substituiertes Phenyl-niedrigalkyl sind, wobei die Substituenten der beiden letzten Gruppen Halogen, Trifluoromethyl, Nitro, Niedrigalkyl oder Niedrigalkoxy sein können;

(ii) wenn $R_2$ und $R_6$ die unter (i) genannten Bedeutungen haben und $R_1$ Wasserstoff oder Methyl, $R_3$ Wasserstoff und $R_5$ Wasserstoff, Niedrigalkyl, unsubstituiertes Phenyl, Benzyl, halogen-substituiertes Benzyl, Hydroxy-niedrigalkyl, Niedrigalkoxy-niedrigalkyl, Phenoxy-niedrigalkyl, Mercapto-niedrigalkyl, Alkylthio-niedrigalkyl, Phenylthio-niedrigalkyl oder Benzyl-niedrigalkyl sind, dann ist $R_4$ ausgewählt aus G—A (wie oben definiert),

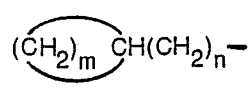

(wie oben definiert, ausgenommen Cyclohexyl und Cyclohexylmethyl), Adamantyl-methyl, Phenyl oder Phenyl-niedrigalkyl, wobei die beiden letztgenannten Gruppen durch Nitro, Niedrigalkyl oder Niedrigalkoxy substituiert sind;

(iii) wenn $R_1$, $R_2$, $R_3$ und $R_5$ die unter (ii) genannten Bedeutungen haben und $R_6$ Amino ist, dann kann $R_4$ nicht die Gruppe —$(CH_2)_t$—$R_x$ sein, worin t 0 oder 1 und $R_x$ Wasserstoff, Alkyl, Cyclohexyl, Phenyl oder durch Halogen, Trifluoromethyl oder Hydroxy substituiertes Phenyl sind;

(iv) wenn $R_6$ Amino, $R_1$ Wasserstoff, $C_1$—$C_{10}$-Alkyl, Hydroxy-niedrigalkyl, Niedrigalkoxy-niedrigalkyl, Aryloxy-niedrigalkyl, Heteroaryloxy-niedrigalkyl, Amino-niedrigalkyl, Niedrigalkylamino-niedrigalkyl, Di-niedrigalkylamino-niedrigalkyl, Acylamino-niedrigalkyl, Arylamino-niedrigalkyl, Guanidino-niedrigalkyl, durch eine heterocyclische Gruppe substituiertes Alkyl, Alkylthio-niedrigalkyl, Arylthio-niedrigalkyl, Aryl-niedrigalkyl, Arylkyloxy-niedrigalkyl, Aralkylthio-niedrigalkyl (wobei die Arylgruppe jedes vorstehend genannten arylhaltigen Substituenten durch Halogen, Niedrigalkyl, Hydroxy, Niedrigalkoxy-amino, Cyano oder Sulfonamido substituiert sein kann), $R_2$ —COOH, —COO-Alkyl, —COO-Aryl, —COO-(Aryl-niedrigalkyl), $R_3$ Wasserstoff und $R_4$ Wasserstoff, Niedrigalkyl, Phenyl-niedrigalkyl, Aminomethylphenyl-niedrigalkyl, Hydroxyphenyl-niedrigalkyl, Hydroxy-niedrigalkyl, Acylamino-niedrigalkyl, Amino-niedrigalkyl, Dimethylamino-niedrigalkyl, Guanidino-niedrigalkyl, Indolylalkyl oder Niedrigalkylthio-niedrigalkyl sind, dann kann $R_5$ keine der Gruppen mit der Struktur

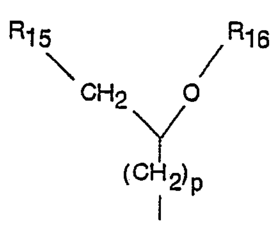 ; 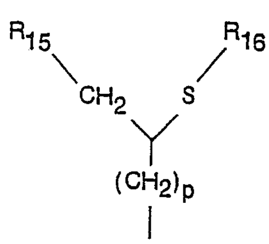 ; 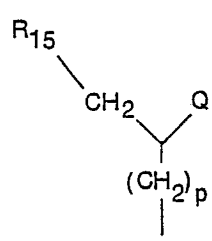 ;

worin

p 0, 1 oder 2,

$R_{15}$ Wasserstoff oder Niedrigalkyl,

$R_{16}$ Niedrigalkyl, Aryl, Aralkyl oder Heteroaryl und

Q $C_3$—$C_8$ Cycloalkyl sind, oder

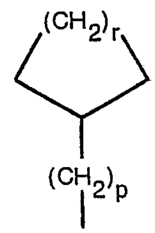

sein, worin p wie oben definiert und r 2 oder 3 sind und worin die Ausdrücke Alkyl, Alkoxy und Alkenyl sich auf Gruppen mit 1—20 Kohlenstoffatomen beziehen, die geradkettig, verzweigtkettig sein oder eine oder mehrere alicyclische Ringe enthalten können und worin Niedrigalkyl und Niedrigalkoxy solche Gruppen mit 1 bis 8 Kohlenstoffatomen bedeuten, dadurch gekennzeichnet, dass sie durch ein geeignetes aus den folgenden ausgewähltes Verfahren (wobei in den folgenden Formeln $R_1$, $R_2$, $R_3$, $R_4$, $R_5$, $R_6$, $R_{10}$, $R_{11}$ und y wie für Formel I definiert sind, einschliesslich geeigneten Schutzes etwaiger reaktiver Gruppen):

a) (für die Herstellung von Verbindung der Formel I, worin $R_3$ Wasserstoff und $R_2$ nicht die Gruppe

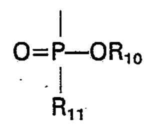

darstellen):
Reduktion einer Verbindung der Formel A

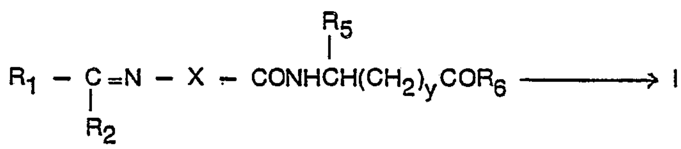

*A*

b) Kuppeln einer Aminosäure A′ mit einer Aminosäure B′

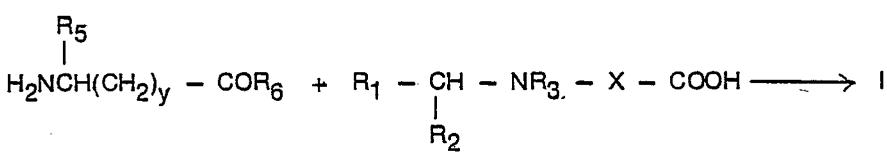

*A′* *B′*

c) (für die Herstellung von Verbindungen der Formel I, worin $R_3$ Wasserstoff ist): Reduktion einer Verbindung der Formel X an der C=N-Doppelbindung

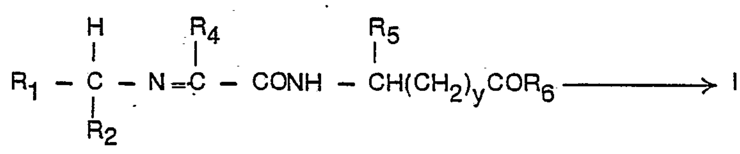

d) Alkylierung eines Amins der Formel S′ mit einer Verbindung der Formel T′

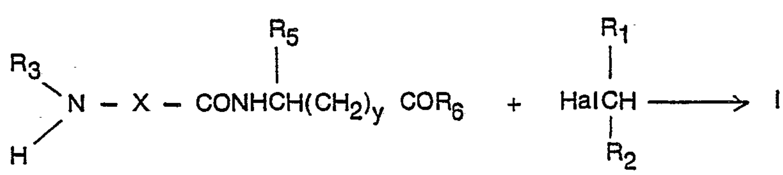

*S′* *T′*

worin Hal Halogen darstellt;

e) (für die Herstellung von Verbindung, in denen $R_3$ Wasserstoff und $R_2$ die Gruppe

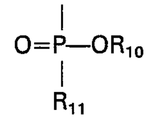

sind): Umsetzung einer Verbindung der Formel "A" mit einem Phosphitester oder Phosphonatester der Formel Z

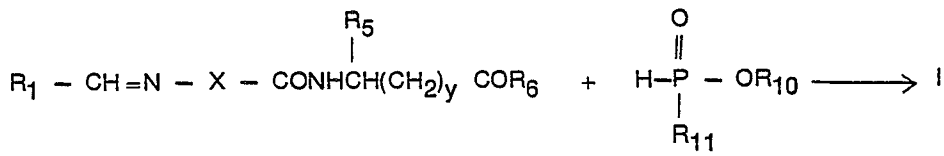

*A''* *Z*

gefolgt von Entfernung etwaiger Schutzgruppen, wenn zur Bildung der gewünschten Verbindung der Formel I nötig, sowie anschliessend gewünschtenfalls von Umwandlung der Verbindung der Formel I in eine andere Verbindung der Formel I und/oder Bildung eines Salzes und gewünschtenfalls Isolation eines bevorzugten Isomeren, wobei die oben verwendeten Ausdrücke Alkyl, Alkoxy und Alkenyl solche Gruppen mit 1 bis 20 Kohlenstoffatomen bedeuten, wobei diese Gruppen gerad- oder verzweigtkettig sein und/oder ein oder mehr alicyclische Ringe enthalten können, und wobei Niedrigalkyl und Niedrigalkoxy solche der vorgenannten Gruppen bedeuten, die 1 bis 8 Kohlenstoffatome enthalten.

2. Verfahren nach Anspruch 1 a) bis d), dadurch gekennzeichnet, dass eine Verbindung hergestellt wird, worin

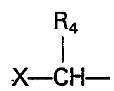

ist, y 1, 2 oder 3 ist, $R_2$ —COOH, —COO-(Niedrigalkyl), —COO(Arylniedrigalkyl) oder —COO-(Aryl) bedeutet, $R_3$ Wasserstoff ist und $R_4$ und $R_5$ aus Wasserstoff, der Gruppe G—A— (wie in Anspruch 1 definiert), 1-Adamantylmethyl, Cycloniedrigalkyl, Hydroxyniedrigalkyl, Alkyl, Acylaminoniedrigalkyl, Amino-niedrigalkyl, Di-niedrigalkylamino-niedrigalkyl, Halogeno-niedrigalkyl, Guanidino-niedrigalkyl, Imidazolylniedrigalkyl, Indolyl-niedrigalkyl, Mercapto-niedrigalkyl, Niedrigalkylthio-niedrigalkyl, unsubstituiertem Phenyl, unsubstituiertem oder substituiertem Phenyl-niedrigalkyl (wobei die Substituenten der beiden letztgenannten Gruppen Halogeno, Niedrigalkyl, Niedrigalkoxy, Trifluoromethyl oder Nitro sein können),

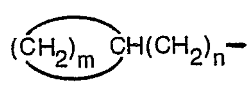

(worin n und m wie in Anspruch 1 definiert sind) und Aminomethyl-phenylniedrigalkyl ausgewählt sind.

3. Verfahren nach Anspruch 1, dadurch gekennzeichnet, dass eine Verbindung hergestellt wird, worin

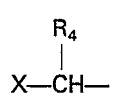

und y null und den Vorbehalten (i), (ii), (iii) und (iv) des Anspruchs 1 unterworfen sind,

$R_1$H, Alkyl, Aralkyl, Phenyl, Aralkyloxyalkyl oder Heteroaryloxyalkyl ist, wobei die drei letztgenannten Gruppen wie in Anspruch 1 definiert substituiert sein können,

$R_3$ Wasserstoff;

$R_4$ aus der Gruppe G—A— (wie in Anspruch 1 definiert), der Gruppe

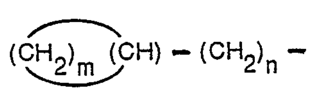

(worin n und m wie in Anspruch 1 definiert sind), substituiertem oder unsubstituiertem Phenyl sowie substituiertem Phenyl-niedrigalkyl ausgewählt ist, wobei die Substituenten der beiden letztgenannten

Gruppen Halogen, Trifluoromethyl, Nitro, Niedrigalkyl oder Niedrigalkoxy sein können, mit dem nur für $R_4$ geltenden Vorbehalt, dass dieses, wenn $R_6$

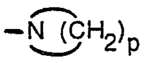 oder 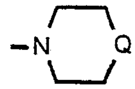

ist, worin p 3, 4 oder 5 und Q Sauerstoff oder Schwefel sind, auch Wasserstoff, Niedrigalkyl, Indolyl-niedrigalkyl, Mercapto-niedrigalkyl, Niedrigalkylthio-niedrigalkyl, Guanidino-niedrigalkyl, Imidazolylniedrigalkyl, hydroxy-substituiertes Phenyl sein kann;

$R_5$ aus den für $R_4$ definierten Gruppen einschliesslich der in dem Vorbehalt genannten; und

$R_6$ aus Hydroxy, Niedrigalkoxy oder der Gruppe $—NR'_8R'_9$ ausgewählt sind, worin $R'_8$ und $R'_9$ unabhängig Wasserstoff oder Niedrigalkyl sind oder zusammen mit dem Stockstoffatom, an das sie gebunden sind, die Gruppe

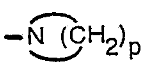 oder 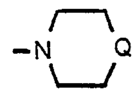

bilden, worin p und Q wie oben definiert sind.

4. Verfahren nach Anspruch 2 oder 3, dadurch gekennzeichnet, dass eine Verbindung hergestellt wird, worin $R_1$ Niedrigalkyl oder Phenyl-niedrigalkyl,

$R_2$ —COOH oder —COO-(Niedrigalkyl),

$R_4$ 2- oder 3-Thienylmethyl, 1- oder 2-Naphthylmethyl, Benzyl oder halogensubstituiertes Benzyl und

$R_6$ —OH oder Niedrigalkoxy sind.

5. Verfahren nach Anspruch 1 a) bis d), dadurch gekennzeichnet, dass eine Verbindung hergestellt wird, worin y 1, 2 oder 3,

X 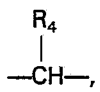

$R_1$ Wasserstoff, Phenyläthyl, Benzyl oder Niedrigalkylthio-niedrigalkyl,

$R_2$ Carboxy oder Niedrigalkoxy-carbonyl,

$R_3$ Wasserstoff,

$R_4$ Benzyl oder Niedrigalkyl,

$R_5$ Wasserstoff und

$R_6$ Hydroxy oder Niedrigalkoxy sind.

6. Verfahren nach Anspruch 1 a) bis d), dadurch gekennzeichnet, dass eine der folgenden Verbindungen:
N-(L-1-Aethoxycarbonyläthyl)-L-2-thienyl-alanyl-glycin,
N-(L-1-Aethoxycarbonyläthyl)-L-2-thienyl-alanyl-L-alanin,
N-(L-1-Aethoxycarbonyläthyl)-L-3-thienyl-alanyl-L-alanin,
N-(L-1-Aethoxycarbonyläthyl)-L-3-thienyl-alanyl-glycin,
N-(L-1-Carboxyäthyl)-L-2-thienylalanyl-L-alanin
oder ein pharmazeutisch annehmbares Salz davon hergestellt wird.

7. Verfahren nach Anspruch 1 a) bis d), dadurch gekennzeichnet, dass eine der folgenden Verbindungen:
N-(L-1-Carboxy-3-phenylpropyl)-L-3-(3-thienyl)alanyl-L-alanin,
N-(D,L-1-Carboxy-3-phenylpropyl)-L-3-(2′-naphthyl)alanyl-L-alanin,
N-(L-1-Aethoxycarbonyl-3-phenylpropyl)-L-3-(2′-thienyl)-alanyl-L-alanin,
N-(D-1-Carboxy-3-phenylpropyl)-L-(4-fluorophenyl)alanyl-L-alanin,
N-(L-1-Carboxy-3-phenylpropyl)-L-3-(1-naphthyl)alanyl-β-alanin,
N-(D-1-Carboxy-3-phenylpropyl)-L-3-(3-thienyl)alanyl-L-alanin,
N-(L-1-Carboxy-2-(benzylthio)-äthyl)-L-phenylalanyl-β-alanin,
N-(L-1-Carboxy-2-phenäthyl)-L-phenylalanyl-β-alanin,
N-(L-1-Carboxy-2-phenäthyl)-L-phenylalanyl-γ-aminobuttersäure,
N-(L-1-Carboxy-3-phenylpropyl)-L-phenylalanyl-β-alanin-Hemihydrat oder
N-(L-1-Carboxy-3-phenylpropyl)-D,L-3-(1-naphthyl)alanyl-L-alanin
oder ein pharmazeutisch annehmbares Salz davon hergestellt wird.

8. Verfahren nach Anspruch 1 b) bis e), dadurch gekennzeichnet, dass eine Verbindung hergestellt wird, worin y null, $R_1$ wie in Anspruch 1 definiert,.

$R_2$ 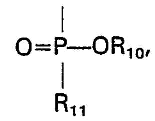

$R_3$, $R_4$, $R_5$, $R_{10}$, $R_{11}$ und X wie in Anspruch 1 definiert und $R_6$ —OH sind, mit dem Vorbehalt, dass

X 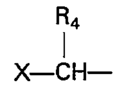

ist, wenn $R_4$ Wasserstoff, Aryl oder Heteroaryl ist.

9. Verfahren nach Anspruch 1, wobei im Verfahren a) eine Ketosäure oder deren Ester der Formel D

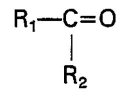 D

mit einer Aminosäure oder deren Ester der Formel C

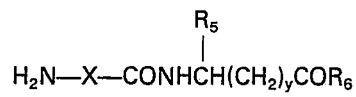 C

zu einer Verbindung der Formel A kondensiert wird, wobei das reaktive Medium ein Reduktionsmittel enthält, wodurch die Verbindung der Formel A, so wie sie durch die obige Kondensation gebildet wird, zur Verbindung der Formel I reduziert wird.

10. Verfahren nach Anspruch 1, wobei im Verfahren c) eine Ketosäure oder deren Ester der Formel E′

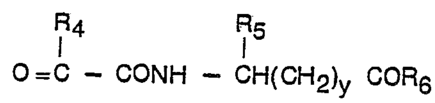 E′

mit einer Aminosäure oder deren Ester der Formel F′

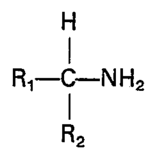 F′

in einem reaktiven Medium, das ein Reduktionsmittel enthält, zur Verbindung der Formel X kondensiert wird, wodurch diese, so wie sie durch die gesamte Kondensation gebildet wird, zur Verbindung der Formel I reduziert wird.

11. Verfahren zur Herstellung einer pharmazeutischen Zubereitung, dadurch gekennzeichnet, dass man eine Verbindung der Formel I, wie in Anspruch 1 definiert, oder ein Salz davon, mit einem pharmazeutisch annehmbaren Trägerstoff oder Exzipienten mischt.

12. Verfahren zur Herstellung einer pharmazeutischen Zubereitung, dadurch gekennzeichnet, dass man eine Verbindung der Formel I oder ein Salz davon, hergestellt nach einem Verfahren nach einem der Ansprüche 1 bis 10, mit einem pharmazeutisch annehmbaren Trägerstoff oder Exzipienten mischt.

**Revendications pour les Etats contractants: BE CH DE FR GB IT LI NL SE**

1. Composé ayant pour formule:

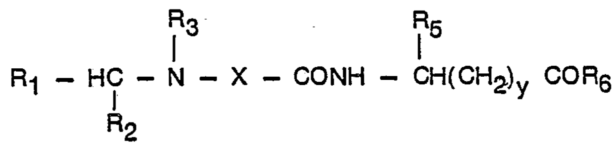

I

et ses sels acceptables en pharmacie, où

$R_1$ est hydrogène; alcoyle; halogène-alcoyle inférieur; hydroxy-alcoyle inférieur; alcoxy inférieur-alcoyle inférieur; aryloxy-alcoyle inférieur; amino-alcoyle inférieur; alcoylamino inférieur-alcoyle inférieur; di-alcoylamino inférieur-alcoyle inférieur; acylamino-alcoyle inférieur; diarylamino-alcoyle inférieure; arylamino-alcoyle inférieur; guanidino-alcoyle inférieur; hétéroaryle; aryle; aralcoyle; mercapto-alcoyle inférieur; arylthio-alcoyle inférieur; alcoyle inférieur-aralcoyle; alcoylthio-alcoyle inférieur; aralcoyloxyalcoyle; aralkylthio-alcoyle; ou hétéroaryloxyalcoyle; où les trois derniers radicaux pouvant être substitués par un halogène, un alcoyle inférieur, un alcoxy inférieur, un hydroxy, un amino, un aminométhyle, un carboxyle, un cyano et/ou un sulfamoyle; ou
alkényle substitué par un groupe hétérocyclique ou alcoyle substitué par un groupe hétérocyclique, ces deux derniers groupes hétérocycliques étant éventuellement substitués par un ou plusieurs groupes choisis parmi alcoyle inférieur, hydroxy, alcoxy inférieur, amino, alcoylamino inférieur, di-alcoylamino inférieur, acylamino, halogéno, halogéno-alcoyle inférieur, cyano et/ou sulfonamide;

$R_2$ est —COOH, COO-(alcoyle inférieur), —COO-(aryl-alcoyle inférieur), —COO aryle, ou bien le groupe

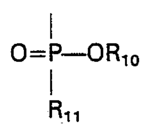 où

$R_{10}$ est hydrogéne, alcoyle inférieur ou benzyle; et
$R_{11}$ est hydroxy, alcoxy, benzyloxy, alcoyle inférieur, aryle ou benzyle;
$R_3$ est hydrogène ou alcoyle inférieur;

X est 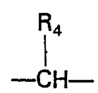 ou 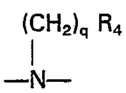

où q est 0 ou 1;

$R_4$ et $R_5$ sont choisis dans les groupes définis pour $R_1$, indolyle (comme 3-indolyle), indolylalcoyle (comme 3-indolylalcoyle), adamantyle (comme 1-adamantyle), adamantylméthyle (comme 1-adamantyl-méthyle) et aminométhylphényl alcoyle inférieur

y est zéro ou un nombre entier de 1 à 3; et

$R_6$ est choisi parmi hydroxy, alcoxy inférieur et le groupe $-NR_8R_9$ où $R_8$ et $R_9$ sont indépendamment de l'hydrogène, un aralcoyle ou un alcoyle inférieur qui, avec l'atome d'azote auquel ils sont attachés, peuvent former un groupe hétérocyclique à 4 à 6 membres, dont l'un des membres peut être de l'oxygène ou du soufre;

et où, quand X est 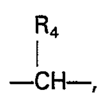,

et y est zéro, les conditions qui suivent s'appliquent:

(i) si $R_2$ est —COOH, —COO (alcoyle inférieur) —COO (aryl -alcoyle inférieur) ou COO aryle et que $R_6$ est —OH, alcoxy inférieur, alcoylamino inférieur, di-alcoylamino inférieur, aralcoylamino ou di-aralcoylamino, alors $R_4$ est choisi parmi G—A— où G est 2- ou 3-thiényle ou 1- ou 2-naphtyle et A est $-CH_2$- ou $-CH_2$-alcoyle inférieur-,

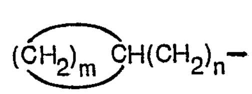

où n est O ou un nombre entier de 1 à 8, et m est un nombre entier de 2 à 8, adamantylméthyle, phényle non substitué ou substitué ou phénylalcoyle inférieur substitué où le(s) substituant(s) de ces deux derniers

groupes est ou sont choisis parmi un halogène, trifluorométhyle, nitro, alcoyle inférieur ou alcoxy inférieur; à condition de plus que

(ii) si, quand $R_2$ et $R_6$ sont tels qu'indiqués à la condition (i), et que $R_1$ est de l'hydrogène ou du méthyle, $R_3$ est de l'hydrogène et $R_5$ est de l'hydrogène, un alcoyle inférieur, un phènyle non substitué, du benzyle, du benzyle substitué par un halogène un hydroxyalcoyle inférieur, un alcoxy inférieur-alcoyle inférieur, un phénoxy-alcoyle inférieur, un mercapto alcoyle inférieur, un alkylthioalcoyle inférieur, un phénylthio-alcoyle inférieur ou du benzyle-alcoyle inférieur, alors $R_4$ soit choisi parmi G—A tel que défini ci-dessus,

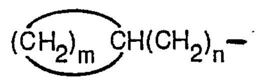

telle que définie ci-dessus, (mais à l'exclusion du cyclohexyle et du cyclohexylméthyle), adamantylméthyle, phényle ou phénylalcoyle inférieur, ces deux derniers groupes étant substitués par nitro, alcoyle inférieur ou alcoxy inférieur;

(iii) si, quand $R_1$, $R_2$, $R_3$ et $R_5$ sont tels qu'indiqués à la condition (ii) et que $R_6$ est amino, alors $R_4$ ne peut être le groupe $—(CH_2)_t—R_x$ où t est 0 ou 1 et $R_x$ est hydrogène, alcoyle, cyclohexyle, phényle ou phényle substitué par un halogène, trifluorométhyle ou hydroxy;

(iv) quand $R_6$ est amino, $R_1$ est hydrogène, alcoyle $C_1—C_{10}$, hydroxy-alcoyle inférieur, alcoxy inférieur-alcoyle inférieur, aryloxy-alcoyle inférieur, hétéroaryloxy-alcoyle inférieur, aminoalcoyle inférieur, alcoyle inférieur-aminoalcoyle inférieur, di-alcoylamino inférieur-alcoyle inférieur, acylaminoalcoyle inférieur, arylaminoalcoyle inférieur, guanidino-alcoyle inférieur, alcoyle substitué par un groupe hétérocyclique, alkylthioalcoyle inférieur, arylthioalcoyle inférieur, arylalcoyle inférieur, aralcoyloxyalcoyle inférieur, aralkylthioalcoyle inférieur où le groupe aryle de tout substituant ci-dessus contenant un aryle peut être substitué par un halogène, un alcoyle inférieur, hydroxy, alcoxy inférieur-amino, cyano ou sulfonamido; $R_2$ est —COOH, —COO alcoyle, COO aryle, —COO (arylalcoyle inférieur), $R_3$ est hydrogène, et $R_4$ est hydrogène, alcoyle inférieur, phénylalcoyle inférieur, amino-méthylphényl-alcoyle inférieur, hydroxy-phénylalcoyle inférieur, hydroxyalcoyle inférieur, acylaminoalcoyle inférieur, aminoalcoyle inférieur, diméthylaminoalcoyle inférieur, guanidinoalcoyle inférieur, indolylalcoyle, alkylthio inférieur-alcoyle inférieur; alors $R_5$ ne peut être un groupe ayant la structure

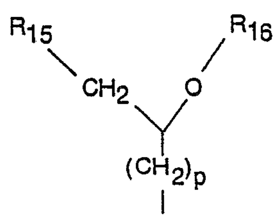 ; 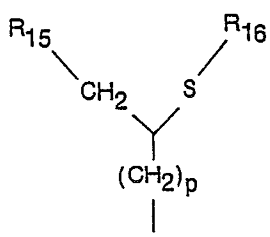 ; 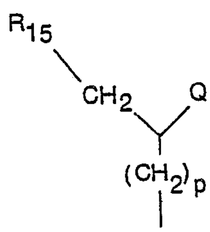 ;

où p est 0, 1 ou 2

$R_{15}$ est hydrogène ou alcoyle inférieur

$R_{16}$ est alcoyle inférieur, aryle, aralcoyle ou hétéroaryle et

Q est cycloalcoyle $C_3—C_8$;

ou le groupe

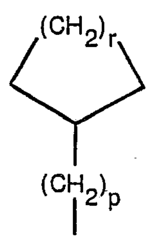

où p est tel que défini ci-dessus et r est 2 ou 3, et où les termes alcoyle, alcoxy et alkényle indiquent de tels groupes ayant de 1 à 20 atomes de carbone, lesdits groupes étant à chaîne droite ou à chaîne ramifiée et/ou comprenant un ou plusieurs noyaux alicycliques; alcoyle inférieur et alcoxy inférieur indiquent les groupes ci-dessus mais ayant de 1 à 8 atomes de carbone.

2. Composé selon la revendication 1, où X est

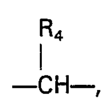

y est 1, 2 ou 3, $R_2$ est —COOH, —COO (alcoyle inférieur), —COO (arylalcoyle inférieur) ou —COO(aryle), $R_3$ est hydrogène et $R_4$ et $R_5$ sont choisis parmi l'hydrogène, le groupe G—A— tel que défini à la revendication 1, 1-adamantylméthyle, cycloalcoyle inférieur, hydroxy-alcoyle inférieur, alcoyle, acylaminoalcoyle inférieur, aminoalcoyle inférieur, di-alcoylamino inférieur-alcoyle inférieur, halogéno alcoyle inférieur, guanidino-alcoyle inférieur, imidazolylalcoyle inférieur, indolylalcoyle inférieur, mercapto-alcoyle inférieur, alkylthio inférieur-alcoyle inférieur, phényle non substitué, phénylalcoyle inférieur non substitué, ou substitué ou le (s) substituant (s) de ces deux derniers groupes est ou sont choisis parmi halogéno, alcoyle inférieur, alcoxy inférieur, trifluorométhyle et nitro,

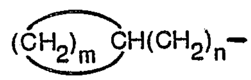

dans laquelle n et m sont tels que définis à la revendication 1, et amino-méthylphényl-alcoyle inférieur.

3. Composé selon la revendication 1, où

X est

$$-\overset{R_4}{\overset{|}{C}H}-,$$

y est zéro, et soumis aux conditions (i), (ii) (iii) et (iv) de la revendication 1,

$R_1$ est H, alcoyle, aralcoyle, phényle; aralkyloxyalcoyle, ou hétéroaryloxyalcoyle où ces trois derniers groupes sont éventuellement substitués comme défini à la revendication 1,

$R_3$ est hydrogène;

$R_4$ est choisi dans le groupe G—A— tel que défini à la revendication 1,

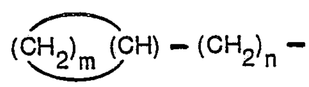

où n et m sont tels que définis à la revendication 1, phényle substitué ou non substitué ou phénylalcoyle inférieur substitué où le (s) substituant (s) de ces deux derniers groupes est ou sont choisis parmi halogène, trifluorométhyle, nitro, alcoyle inférieur et alcoxy inférieur, à condition, pour $R_4$ seulement, que quand $R_6$ est le groupe

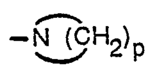 ou 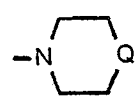

où p est 3, 4 ou 5 et Q est oxygène ou soufre, $R_4$ puisse également être hydrogène, alcoyle inférieur, indolylalcoyle inférieur, mercapto alcoyle inférieur, alkylthio inférieur-alcoyle inférieur, guanidinoalcoyle inférieur, imidazolylalcoyle inférieur, phényle substitué en hydroxy,

$R_5$ est choisi dans les groupes définis pour $R_4$ ci-dessus, comprenant ceux indiqués dans les conditions de cette revendication, et

$R_6$ est choisi parmi hydroxy, alcoxy inférieur ou le groupe —$NR'_8R'_9$, où $R'_8$ $R'_9$ sont indépendamment choisis parmi un hydrogène, un alcoyle inférieur ou, avec l'atome d'azote auquel ils sont attachés, sont

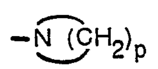 ou 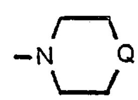

p et Q sont tels que définis ci-dessus.

4. Composé selon la revendication 2 ou 3, où $R_1$ est choisi parmi alcoyle inférieur et phénylalcoyle inférieur,

$R_2$ est choisi parmi —COOH et —COO-(alcoyle inférieur),

$R_4$ est choisi parmi 2- ou 3-thiénylméthyle, 1- ou 2-naphtylméthyle, benzyle ou benzyle substitué en halogéno, et

$R_6$ est —OH ou alcoxy inférieur.

5. Composé selon la revendication 1, où y est 1, 2 ou 3,

X est

$$-\overset{R_4}{\overset{|}{C}H}-$$

$R_1$ est hydrogène, phényléthyle, benzyle ou alkylthio inférieur alcoyle inférieur,

$R_2$ est carboxy ou alcoxy inférieur carbonyle,

$R_3$ est hydrogène,
$R_4$ est benzyle ou alcoyle inférieur,
$R_5$ est hydrogène et
$R_6$ est hydroxy ou alcoxy inférieur.

6. Composé selon la revendication 1, le composé étant
N-(L-1-éthoxycarbonyléthyl)-L-2-thiényl-alanyl-glycine,
N-(L-1-éthoxycarbonyléthyl)-L-2-thiényl-alanyl-L-alanine,
N-(L-1-éthoxycarbonyléthyl)-L-3-thiényl-alanyl-L-alanine,
N-(L-1-éthoxycarbonyléthyl)-L-3-thiényl-alanyl-glycine,
N-(L-1-carboxyéthyl)-L-2-thiénylalanyl-L-alanine
ou un sel acceptable en pharmacie.

7. Composé selon la revendication 1, le composé étant
N-(L-1-carboxy-3-phénylpropyl)-L-3-(3-thiényl)alanyl-L-alanine
N-(D,L-1-carboxy-3-phénylpropyl)-L-3-(2'-naphtyl)alanyl-L-alanine
N-(L-1-éthoxycarbonyl-3-phénylpropyl)-L-3-(2'-thiényl)-alanyl-L-alanine
N-(D-1-carboxy-3-phénylpropyl)-L-(4-fluorophényl)alanyl-L-alanine
N-(L-1-carboxy-3-phénylpropyl)-L-3-(1-naphtyl)alanyl-β-alanine,
N-(D-1-carboxy-3-phénylpropyl)-L-3-(3-thiényl)alanyl-L-alanine
N-(L-1-carboxy-2-(benzylthio)-éthyl-L-phénylalanyl-β-alanine
N-(L-1-carboxy-2-phénéthyl)-L-phénylalanyl-β-alanine Acide N-(L-1-carboxy-2-phénéthyl)-L-phénylalanyl-δ-aminobutyrique
N-(L-1-carboxy-3-phénylpropyl)-L-phénylalanyl-β-alanine hémihydrate ou
N-(L-1-carboxy-3-phénylpropyl)-D,L-3-(1-naphtyl)alanyl-L-alanine
ou un sel acceptable en pharmacie.

8. Composé selon la revendication 1, où y est zéro, $R_1$ est tel que défini à la revendication 1,

$R_2$ est 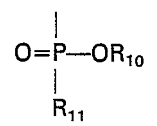

$R_3$, $R_4$, $R_5$, $R_{10}$ et $R_{11}$ et X sont tels que définis à la revendication 1, $R_6$ est —OH,
à condition que, quand $R_4$ est hydrogène, aryle ou hétéroaryle,

X soit 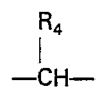

9. Composition pharmaceutique comprenant un composé de formule I tel que défini à la revendication 1, ou son sel, en mélange avec un véhicule ou excipient acceptable en pharmacie.

10. Composition pharmaceutique selon la revendication 9 où le composé est
N-(L-1-carboxy-3-phénylpropyl)-D,L-3-(1-naphtyl)alanyl-L-alanine, ou
N-(L-1-carboxy-3-phénylpropyl)-L-phényl-alanyl-β-alanine hémihydrate.

11. Composé de formule I tel que défini à la revendication 1 comprenant les composés exclus de la revendication 1 par la condition (i) mais à l'exclusion des composés exclus de la revendication 1 par les conditions (ii), (iii) ou (iv), pour une utilisation dans l'inhibition de l'encéphalinase.

**Revendications pour l'Etat contractant: AT**

1. Procéde de préparation d'un composé ayant pour formule:

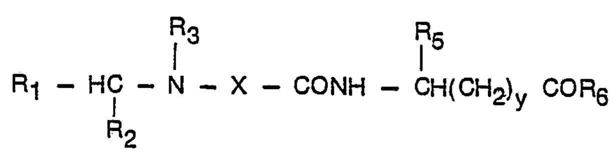 I

où

$R_1$ est hydrogène; alcoyle; halogène-alcoyle inférieur; hydroxy-alcoyle inférieur; alcoxy inférieur-alcoyle inférieur; aryloxy-alcoyle inférieur; amino-alcoyle inférieur; alcoylamino inférieur-alcoyle inférieur; di-alcoylamino inférieur-alcoyle inférieur; acylamino-alcoyle inférieur; diarylamino-alcoyle inférieure arylamino-alcoyle inférieur; guanidino-alcoyle inférieur; hétéroaryle; aryle; aralcoyle; mercapto-alcoyle inférieur; arylthio-alcoyle inférieur; alcoyle inférieur-aralcoyle; alcoylthio-alcoyle inférieur; aralcoyloxyalcoyle; aralkylthio-alcoyle; ou hétéroaryloxyalcoyle; les trois derniers radicaux pouvant être substitués par un halogène, un alcoyle inférieur, un alcoxy inférieur, un hydroxy, un amino, un

aminométhyle, un carboxyle, un cyano et/ou un sulfamoyle; ou alkényle substitué par un groupe hétérocyclique ou alcoyle substitué par un groupe hétérocyclique, ces deux derniers groupes hétérocycliques étant éventuellement substitués par un ou plusieurs groupes choisis parmi alcoyle inférieur, hydroxy, alcoxy inférieur, amino, alcoylamino inférieur, di-alcoylamino inférieur, acylamino, halogéno, halogéno-alcoyle inférieur, cyano et/ou sulfonamide;

$R_2$ est —COOH, COO-(alcoyle inférieur), —COO-(aryl-alcoyle inférieur), —COO (aryle), ou bien le groupe

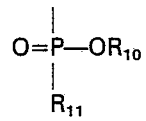 où

$R_{10}$ est hydrogéne, alcoyle inférieur ou benzyle; et

$R_{11}$ est hydroxy, alcoxy, benzyloxy, alcoyle inférieur, aryle ou benzyle;

$R_3$ est hydrogène ou alcoyle inférieur;

X est 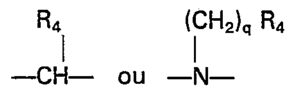

où q est 0 ou 1;

$R_4$ et $R_5$ sont choisis dans les groupes définis pour $R_1$, indolyle (comme 3-indolyle), indolylalcoyle (comme 3-indolylalcoyle), adamantyle (comme 1-adamantyle), adamantyl-méthyle (comme 1-adamantyl-méthyle) et aminométhylphénylalcoyle inférieur

y est zéro ou un nombre entieur de 1 à 3; et

$R_6$ est choisi parmi hydroxy, alcoxy inférieur et le groupe —$NR_8R_9$ où $R_8$ et $R_9$ sont indépendamment de l'hydrogène, un aralcoyle ou un alcoyle inférieur qui, avec l'atome d'azote auquel ils sont attachés, peuvent former un groupe hétérocyclique à 4 à 6 membres, dont l'un des membres peut être de l'oxygène ou du soufre;

et où, quand X est 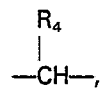

et y est zéro, les conditions qui suivent s'appliquent:

(i) si $R_2$ est —COOH, —COO (alcoyle inférieur), —COO(aryl -alcoyle inférieur) ou COO aryle et $R_6$ est —OH, alcoxy inférieur, alcoyle inférieur-amino, di-alcoyle inférieur-amino, aralcoyl-amino ou diaralcoylamino, alors $R_4$ est choisi parmi G—A— où G est 2- ou 3-thiényle ou 1- ou 2-naphtyle et A est —$CH_2$— ou —$CH_2$-alcoyle inférieur-,

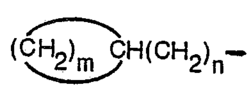

où n est zéro ou un nombre entier de 1 à 8 et m est un nombre entier de 2 à 8, adamantylméthyle, phényle non substitué ou substitué ou phénylalcoyle inférieur substitué où le(s) substituant(s) de ces deux derniers groupes est ou sont choisis parmi halogène, trifluorométhyle, nitro, alcoyle inférieur ou alcoxy inférieur; à condition de plus que

(ii) si, lorsque $R_2$ et $R_6$ sont tels qu'indiqués à la condition (i), et $R_1$ est de l'hydrogène ou du méthyle, $R_3$ est de l'hydrogéne et $R_5$ est de l'hydrogène, un alcoyle inférieur, du phényle non substitue, du benzyle, du benzyle substitué par un halogène, un hydroxy-alcoyle inférieur, un alcoxy inférieur-alcoyle inférieur, un phénoxyalcoyle inférieur, un mercapto-alcoyle inférieur, un alkylthio-alcoyle inférieur, un phénylthio-alcoyle inférieur ou un benzyl-alcoyle inférieur, alors $R_4$ soit choisi parmi G—A tel que défini ci-dessus

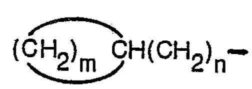

telle que définie ci-dessus, (mais à l'exclusion du cyclohexyle et du cyclohexylméthyle) adamantylméthyle, phényle ou phénylalcoyle inférieur, ces deux derniers groupes étant substitués par nitro, alcoyle inférieur ou alcoxy inférieur;

(iii) si, quand $R_1$, $R_2$, $R_3$ et $R_5$ sont tels qu'indiqués à la condition (ii) et que $R_6$ est amino, alors $R_4$ ne peut être le groupe —$(CH_2)_t$—$R_x$ où t est O ou 1 et $R_x$ est hydrogéne, alcoyle, cyclohexyle, phényle ou phényle substitué par un halogène, trifluorométhyle ou hydroxy;

(iv) quand $R_6$ est amino, $R_1$ est hydrogène, alcoyle $C_1$—$C_{10}$, hydroxy-alcoyle inférieur, alcoxy inférieur-alcoyle inférieur, aryloxy-alcoyle inférieur, hétéroaryloxy-alcoyle inférieur, amino-alcoyle inférieur, alcoyle

inférieur-aminoalcoyle inférieur, di-alcoylamino inférieur-alcoyle inférieur, acylamino-alcoyle inférieur, arylamino-alcoyle inférieur, guanidino-alcoyle inférieur, alcoyle substitué par un groupe hétérocyclique, alkylthio-alcoyle inférieur, arylthio-alcoyle inférieur, arylalcoyle inférieur, aralcoyloxy-alcoyle inférieur, aralkylthio-alcoyle inférieur où le groupe aryle de tout substituant ci-dessus contenant un groupe aryle peut être substitué par un halogène, un alcoyle inférieur, hydroxy, alcoxyamino inférieur, cyano ou sulfonamido, $R_2$ est —COOH, —COO alcoyle, COO aryle, —COO (arylalcoyle inférieur), $R_3$ est hydrogène et $R_4$ est hydrogène, alcoyle inférieur, phénylalcoyle inférieur, amino-méthylphényl-alcoyle inférieur, hydroxy-phénylalcoyle inférieur, hydroxy-alcoyle inférieur, acylaminoalcoyle inférieur, amino-alcoyle inférieur, diméthylamino-alcoyle inférieur, guanidino-alcoyle inférieur, indolylalcoyle, alkylthio-inférieur-alcoyle inférieur; alors

$R_5$ ne peut être un groupe ayant la structure

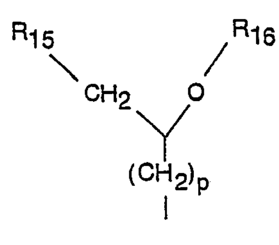 ; 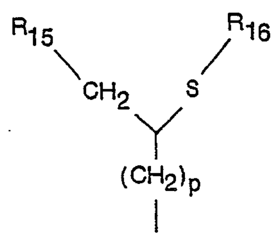 ; 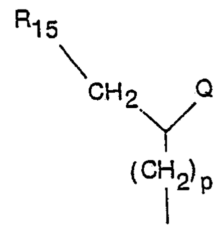 ;

où p est 0, 1 ou 2

$R_{15}$ est hydrogène ou alcoyle inférieur

$R_{16}$ est alcoyle inférieur, aryle, aralcoyle ou hétéroaryle et

Q est cycloalcoyle $C_3$—$C_8$;

ou le groupe

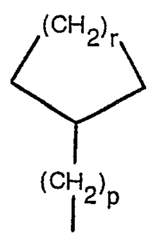

où p est tel que défini ci-dessus et r est 2 ou 3, ou un sel acceptable en pharmacie, caractérisé en ce que le composé est préparé par une méthode appropriée choisie parmi les méthodes qui suivent (où, dans les formules qui suivent $R_1$, $R_2$, $R_3$, $R_4$, $R_5$, $R_6$, $R_{10}$, $R_{11}$ et y sont tels que définis pour la formule I, comprenant une protection appropriée de tout groupe réactif):

a) (Pour la préparation de composés de formule I où $R_3$ est de l'hydrogène et $R_2$ est autre que

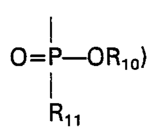

la réduction d'un composé de formule A

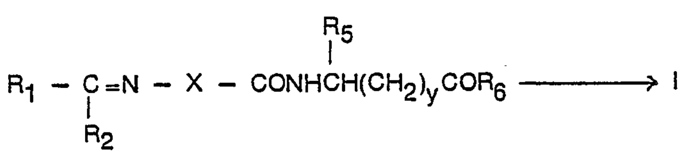

*A*

b) Le couplage d'un aminoacide A' avec un aminoacide B'

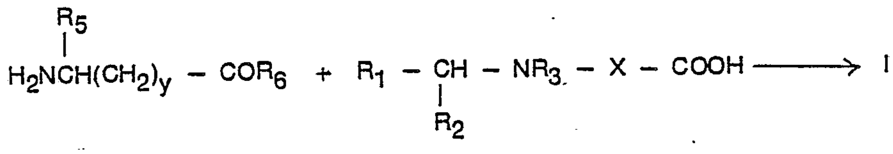

A'　　　B'

c) (Pour la préparation de composés de formule I où $R_3$ est de l'hydrogène), la réduction d'un composé de formule X, à la double liaison C=N,

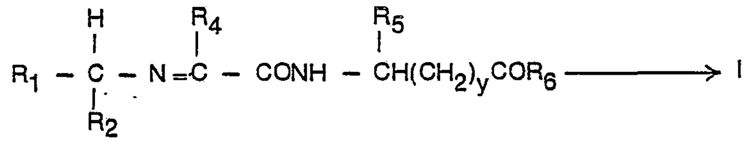

d) L'alcoylation d'une amine de formule S' avec un composé de formule T'

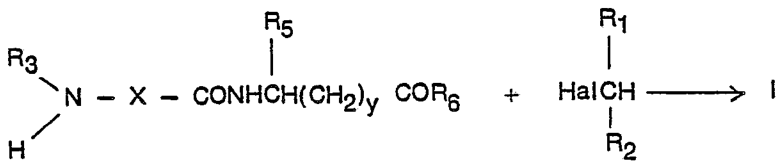

S'　　　T'

où Hal représente un halogène

e) (Pour la préparation de composés où $R_3$ est de l'hydrogène et $R_2$ est

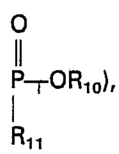

la réaction d'un composé de formule A'' avec un phosphite ester ou phosphonate ester de formule Z

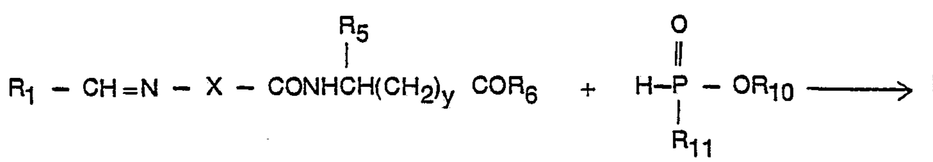

A''　　　Z

avec ensuite enlèvement de tout groupe protecteur si nécessaire pour obtenir le composé souhaité de formule I, et après, si on le souhaite, le conversion du composé de formule I en un autre composé de formule I et/ou la formation de son sel, et si on le souhaite, l'isolement d'un isomère préféré; et où les termes alcoyle, alcoxy et alkènyle utilisés ci-dessus indiquent ces groupes ayant de 1 à 20 atomes de carbone, lesdits groupes étant à chaîne droite, ou à chaîne ramifiée et/ou comprenant un ou plusieurs noyaux alicycliques; alcoyle inférieur et alcoxy inférieur indiquent ces groupes mais ayant de 1 à 8 atomes de carbone.

2. Procédé selon la revendication 1 a) à d), caractérisé en ce qu'un composé est produit dans lequel

X est 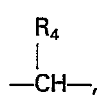

y est 1, 2 ou 3, $R_2$ est —COOH, —COO (alcoyle inférieur), —COO (arylalcoyle inférieur) ou —COO (aryle), $R_3$ est hydrogène et $R_4$ et $R_5$ sont choisis parmi l'hydrogène, le groupe G—A— tel que défini à la

revendication 1, 1-adamantylméthyle, cycloalcoyle inférieur, hydroxyalcoyle inférieur, alcoyle, acylaminoalcoyle inférieur, aminoalcoyle inférieur, di-alcoylamino inférieur-alcoyle inférieur, halogéno-alcoyle inférieur, guanidino-alcoyle inférieur, imidazolylalcoyle inférieur, indolylalcoyle inférieur, mercapto-alcoyle inférieur, alkylthio inférieur-alcoyle inférieur, phényle non substitué, phényl-alcoyle inférieur non substitué ou substitué ou le(s) substituant(s) de ces deux, derniers groupes est ou sont choisis parmi halogéno, alcoyle inférieur, alcoxy inférieur, trifluorométhyle et nitro,

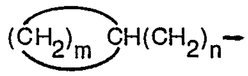

où n et m sont tels que définis à la revendication 1, et aminométhylphényl-alcoyle inférieur.

3. Procédé selon la revendication 1, caractérisé en ce qu'un composé est produit dans lequel

X est 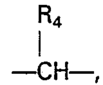

y est zéro et soumis aux conditions (i), (ii), (iii) et (iv) de la revendication 1.

$R_4$ est H, alcoyle, aralcoyle, phényle, aralcoyloxy-alcoyle ou hétéroaryloxyalcoyle, où ces trois derniers groupes sont éventuellement substitués comme défini à la revendication 1,

$R_3$ est hydrogène;

$R_4$ est choisi dans le groupe G—A tel que défini à la revendication 1,

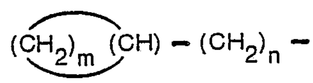

où n et m sont tels que définis à la revendication 1, phényle substitué ou non substitué ou phényle-alcoyle inférieur substitué ou le(s) substituant(s) de ces deux derniers groupes est ou sont choisis parmi halogène trifluorométhyle, nitro, alcoyle inférieur et alcoxy inférieur, à condition pour $R_4$ seulement que lorsque $R_6$ est le groupe

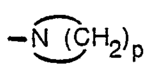 ou 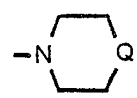

où p est 3, 4 ou 5, et Q est de l'oxygène ou du soufre, $R_4$ puisse également être de l'hydrogène, alcoyle inférieur, indolylalcoyle inférieur, mercapto-alcoyle inférieur, alcoylthio inférieur-alcoyle inférieur, guanidino-alcoyle inférieur, imidazolyl-alcoyle inférieur, phényle substitué par un hydroxy,

$R_5$ est choisi dans les groupes définis pour $R_4$ ci-dessus comprenant ceux indiqués dans la condition de cette revendication et,

$R_6$ est choisi parmi hydroxy, alcoxy inférieur ou le groupe $—NR'_8R'_9$, où $R'_8$ et $R'_9$ sont indépendamment choisis parmi l'hydrogène, un alcoyle inférieur ou, avec l'atome d'azote auquel ils sont attachés, sont

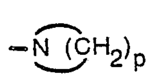 ou 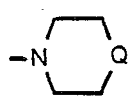

où p et Q sont tels que définis ci-dessus.

4. Procédé selon la revendication 2 ou 3, caractérisé en ce qu'un composé est produit dans lequel $R_1$ est choisi parmi un alcoyle inférieur et un phénylalcoyle inférieur,

$R_2$ est choisi parmi —COOH et —COO-(alcoyle inférieur),

$R_4$ est choisi parmi le 2- ou 3-thiénylméthyle, le 1- ou 2-naphtylméthyle, du benzyle ou du benzyle substitué par un halogéno, et

$R_6$ est —OH ou alcoxy inférieur.

5. Procédé selon la revendication 1 a) à d) caractérisé en ce qu'un composé est produit où y est 1, 2 ou 3,

X est 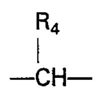

$R_1$ est hydrogène, phényléthyle, benzyle ou alkylthio inférieur-alcoyle inférieur,

$R_2$ est carboxy ou alcoxy inférieur carbonyle,

$R_3$ est hydrogène,
$R_4$ est benzyle ou alcoyle inférieur,
$R_5$ est hydrogène et
$R_6$ est hydroxy ou alcoxy inférieur

6. Procédé selon la revendication 1 a) à d) caractérisé en ce que le composé
N-(L-1-éthoxycarbonyléthyl)-L-2-thiényl-alanyl-glycine,
N-(L-1-éthoxycarbonyléthyl)-L-2-thiényl-alanyl-L-alanine,
N-(L-1-éthoxycarbonyléthyl)-L-3-thiényl-alanyl-L-alanine,
N-(L-1-éthoxycarbonyléthyl)-L-3-thiényl-alanyl-glycine,
N-(L-1-carboxyéthyl)-L-2-thiénylalanyl-L-alanine
ou un sel acceptable en pharmacie est produit.

7. Procédé selon la revendication 1 a) à d) caractérisé en ce que le composé
N-(L-1-carboxy-3-phénylpropyl)-L-3-(3-thiényl)alanyl-L-alanine
N-(D,L-1-carboxy-3-phénylpropyl)-L-3-(2'-naphtyl)alanyl-L-alanine
N-(L-1-éthoxycarbonyl-3-phénylpropyl)-L-3-(2'-thiényl)-alanyl-L-alanine
N-(D-1-carboxy-3-phénylpropyl)-L-(4-fluorophényl)alanyl-L-alanine
N-(L-1-carboxy-3-phénylpropyl)-L-3-(1-naphtyl)alanyl-β-alanine,
N-(D-1-carboxy-3-phénylpropyl)-L-3-(3-tiényl)alanyl-L-alanine
N-(L-1-carboxy-2-(benzylthio)-éthyl-L-phénylalanyl-β-alanine
N-(L-1-carboxy-2-phénéthyl)-L-phénylalanyl-β-alanine Acide N-(L-1-carboxy-2-phénéthyl)-L-phénylalanyl-δ-aminobutyrique
N-(L-1-carboxy-3-phénylpropyl)-L-phénylalanyl-β-alanine hémihydrate ou
N-(L-1-carboxy-3-phénylpropyl)-D,L-3-(1-naphtyl)alanyl-L-alanine
ou son sel acceptable en pharmacie est produit.

8. Procédé selon la revendication 1 b) à e) caractérisé en ce qu'un composé est produit dans lequel y est zéro,
$R_1$ est tel que défini à la revendication 1,

$R_2$ est

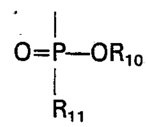

$R_3$, $R_4$, $R_5$, $R_{10}$ et $R_{11}$ et X sont tels que définis à la revendication 1, $R_6$ est —OH, à condition que, quand $R_4$ est de l'hydrogène, de l'aryle ou un hétéroaryle,

X soit

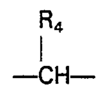

9. Procédé selon la revendication 1, où le procédé a) consiste à condenser un cétoacide ou ester de formule D

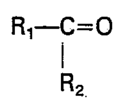

D

avec un aminoacide ou ester de formule C

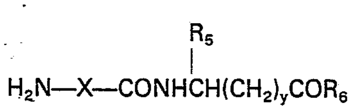

C

pour former le composé de formule A, dans un milieu réactif contenant un agent réducteur afin d'effectuer la réduction du composé de formule A en composé de formule I dans ledit milieu réactif tandis qu'il s'y forme par la condensation ci-dessus.

10. Procédé selon la revendication 1, où le procédé c) consiste à condenser un cétoacide ou ester de formule E'

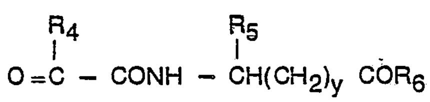

E'

avec un aminoacide ou ester de formule F'

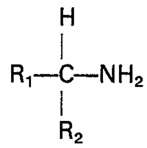

F'

pour former le composé de formule X, dans un milieu réactif contenant un agent réducteur afin d'effectuer la réduction du composé de formule X en composé de formule I dans ledit milieu réactif tandis qu'il se forme par la condensation ci-dessus.

11. Procédé de production d'une composition pharmaceutique caractérisé en ce qu'il consiste à mélanger un composé de formule I tel que défini à la revendication 1, ou son sel, avec un véhicule ou excipient acceptable en pharmacie.

12. Procédé de production d'une composition pharmaceutique caractérisé en ce qu'il consiste à mélanger un composé de formule I ou son sel, préparé par le procéde selon l'une quelconque des revendications 1 à 10, avec un véhicule ou excipient acceptable en pharmacie.